# EUROPÄISCHE PATENTSCHRIFT

(11) **EP 4 103 085 B1**
(45) Veröffentlichungstag und Bekanntmachung des Hinweises auf die Patenterteilung: **06.11.2024**
(21) Anmeldenummer: 20708436.9
(22) Anmeldetag: 14.02.2020
(51) Int. Cl.: A61B 17/86, A61B 50/00, A61B 90/94, A61B 50/20, A61B 50/22, A61B 50/30

(54) **TRÄGER FÜR CHIRURGISCHE GEGENSTÄNDE SOWIE SETS UND CONTAINER MIT SOLCHEN TRÄGERN**
SUPPORTS FOR SURGICAL ITEMS AS WELL AS SETS AND CONTAINERS COMPRISING SUCH SUPPORTS
SUPPORTS POUR ARTICLES CHIRURGICAUX, ENSEMBLES ET RÉCIPIENTS COMPRENANT DE TELS SUPPORTS

(43) Veröffentlichungstag der Anmeldung: 21.12.2022
(73) Patentinhaber: Medartis Holding AG, 4057 Basel (CH)
(72) Erfinder: PFEFFERLE, Raphael, 79244 Münstertal (DE); BRAND, Stefan, Bern (CH); KERN, Philippe Robert, 79692 Kleines Wiesental (DE); MÜLLER, Marco Andreas, 4421 St. Pantaleon (CH); POCHLATKO, Nikolaus, 4144 Arlesheim (CH); SCHONHARDT, Jürgen, 79618 Rheinfelden (DE); STEFFANONI, Seline, 7500 St. Moritz (CH); ZEUNER, Hermann, 79111 Freiburg (CH)
(74) Vertreter: Hepp Wenger Ryffel AG
(86) Internationale Anmeldenummer: PCT/EP2020/053824
(87) Internationale Veröffentlichungsnummer: WO 2021/160274

(56) Entgegenhaltungen:
- EP-A2- 1 842 505
- EP-A2- 3 181 060
- WO-A2-2009/024189
- DE-A1- 102004 046 984
- DE-B3- 102007 011 093
- US-A1- 2008 302 688
- US-A1- 2009 266 728

## Beschreibung

Die vorliegende Erfindung befasst sich mit Trägern zur Aufnahme von chirurgischen Gegenständen, und zwar unter anderem von Implantaten wie beispielsweise Knochenplatten oder von Knochenschrauben. Des Weiteren befasst sie sich mit Sets und Containern, die derartige Träger enthalten.

Gattungsgemässe Träger zur Aufnahme von Knochenplatten sind beispielsweise im Dokument WO 2009/024189 offenbart. Die Träger weisen Stützbereiche auf, die im Bereich einer Öffnung der Knochenplatte liegen. Ein Halteelement ist aus einem thermoplastischen Kunststoff und elastisch ausgebildet. Die Knochenplatte wird von den Schnappelementen seitlich gehalten und in Richtung einer Grundfläche gedrückt. Die Knochenplatte ist derart mit den Halteelementen in Kontakt, dass kein Kontakt zwischen der Knochenplatte und der Grundfläche besteht.

Die in WO 2010/097447 offenbarten Knochenplattenhalter sind derart gestaltet, dass sie übereinander gestapelt werden können. Die Knochenplattenhalter weisen jeweils eine Öffnung auf, in welche die Knochenplatten seitlich eingeschoben werden können. Obwohl die aus dem Stand der Technik bekannten Träger für Knochenplatten bereits eine Reihe von Vorteilen aufweisen, sind Weiterentwicklungen wünschenswert, die das Einsetzen und Entnehmen der Knochenplatte vereinfachen.

Auch gattungsgemässe Träger für Knochenschrauben sind aus dem bereits genannten Dokument WO 2010/097447 bekannt. Die Schraube wird an einer Unterseite ihres Kopfes durch eine Stützfläche des Trägers gehalten. Zusätzlich wird die Schraube durch elastische Zungen gesichert, welche am Gewinde der Schraube anliegen. Weiterhin offenbart das Dokument EP 2 392 286 einen Träger, der eine Aufnahmeöffnung aufweist, in welche eine Schraube eingesetzt ist. Zusätzlich weist der Träger zur Sicherung eine Haltezunge auf. In der aufgenommenen Position sichert die Haltezunge die Schraube.

Weiterhin offenbart die DE 10 2004 046984 A1 einen gattungsgemäßen Träger zur Aufnahme zumindest eines chirurgischen Gegenstandes. Dieser bekannte Träger enthält eine Halterung mit zumindest einer Aufnahme für den zumindest einen chirurgischen Gegenstand, ein Sicherungselement für den zumindest einen Gegenstand. Das Sicherungselement weist zumindest eine Ausnehmung auf. Das Sicherungselement ist bewegbar mit der Halterung verbunden, sodass das Sicherungselement von einer ersten Position in eine zweite Position bewegbar ist und in der ersten Position des Sicherungselements relativ zur Halterung der zumindest eine Gegenstand gesichert ist.

Auch hinsichtlich der Träger für Knochenschrauben sind Weiterentwicklungen wünschenswert, die beispielsweise eine noch bessere Sicherung der Schraube im Träger oder eine einfachere Überprüfung der Sicherung ermöglichen.

Es stellt sich somit die Aufgabe, die aus dem Stand der Technik bekannten Träger für chirurgische Gegenstände, insbesondere für Implantate wie etwa Knochenplatten oder für Knochenschrauben, weiterzuentwickeln - insbesondere dahingehend, dass die chirurgischen Gegenstände einfacher eingesetzt und entnommen werden können und/oder dass sie besser gegen ein Herausfallen gesichert sind und/oder dass die Sicherung leicht zu überprüfen ist. Nachfolgend wird unter einer Sicherung des chirurgischen Gegenstandes stets das Verhindern des Herausfallens des chirurgischen Gegenstandes aus dem Träger verstanden.

Die erfindungsgemäße Lösung liegt in den Merkmalen des unabhängigen Anspruchs 1. Vorteilhafte Weiterbildungen der Erfindung sind Gegenstand der abhängigen Ansprüche.

In der vorliegenden Offenbarung werden auch Beispiele beschrieben, die keine Ausführungsformen der Erfindung sind. Sie dienen lediglich dem Verständnis der Erfindung.

In einem ersten Beispiel betrifft die vorliegende Offenbarung einen Träger zur Aufnahme eines chirurgischen Implantats, insbesondere einer Knochenplatte. Der Träger enthält eine Implantataufnahme, die im Wesentlichen länglich entlang einer Längsachse ausgebildet ist und einen ersten und einen zweiten Teil aufweist. Der erste Teil weist einen Schaft zur Aufnahme des chirurgischen Implantats an einer Ausnehmung des Implantats auf, und der zweite Teil weist einen ersten Befestigungsabschnitt zur Befestigung an einem Halteabschnitt eines Containers auf. Optional kann der erste Befestigungsabschnitt auch zur Positionierung am genannten Halteabschnitt dienen. Überdies enthält der Träger mindestens ein elastisches Element, insbesondere eine Feder, zur Sicherung des Implantats.

Gemäss dem ersten Beispiel ist das elastische Element ausgebildet, durch eine Kraftkomponente im Wesentlichen senkrecht zur Längsachse ein an der Implantataufnahme gehaltenes Implantat zu sichern. Die Ausbildung des elastischen Elements erlaubt ein schnelles Sichern und eine einfache Entnahme des Implantats. Zudem ist die Implantataufnahme für verschiedene Implantatgrössen geeignet und daher flexibel einsetzbar.

In einigen Ausführungsformen ist es möglich, dass die Implantataufnahme und das elastische Element übereinstimmen. In diesen Ausführungsformen bildet also die Implantataufnahme selbst ein elastisches Element. Beispielsweise kann die Implantataufnahme zwei Blattfedern enthalten, die sich im Wesentlichen entlang einer gemeinsamen Längsachse erstrecken, die jedoch in einem mittleren Bereich einen grösseren Abstand voneinander aufweisen als in ihren beiden Endbereichen.

Der Schaft der Implantataufnahme kann derart dimensioniert sein, dass nur ein einziges Implantat aufgenommen werden kann. In vielen Fällen ist es jedoch sinnvoll, wenn der Schaft derart dimensioniert ist, dass mehrere Implantat aufgenommen werden können, beispielsweise indem diese übereinander gestapelt werden. Bevorzugt ist das elastische Element als Verlustsicherung ausgebildet. Mit anderen Worten ist es so ausgebildet, dass es ein unbeabsichtigtes Herausfallen des Implantats aus dem Träger verhindert, beispielsweise durch eine weiter unten noch detailliert beschriebene Klemmung.

Vorzugsweise streckt sich das elastische Element im Wesentlichen entlang der Längsachse der Implantataufnahme und ist zumindest senkrecht zu dieser Richtung elastisch. Dabei ist das elastische Element so angeordnet, dass es bei einer Entnahme des chirurgischen Implantats elastisch verformt wird. Diese Ausgestaltung bewirkt ein Einklemmen des chirurgischen Implantats in der genannten senkrechten Richtung, verhindert jedoch nicht dessen Entnahme in Richtung der Längsachse. Nach Entnahme des chirurgischen Implantats wird die Verformung des elastischen Elements wieder aufgehoben.

Mit weiterem Vorteil erstreckt sich die Implantataufnahme entlang der Längsachse in Richtung des ersten Teiles über das elastische Element hinaus. Hierdurch kann der Träger leicht an der Implantataufnahme ergriffen werden, und das Implantat kann leicht auf die Implantataufnahme geführt oder gesetzt werden.

Weiterhin bevorzugt ist es, wenn die Implantataufnahme auf der Seite des ersten Teiles ein gerundetes Ende zur Aufnahme des Implantats aufweist. Hierdurch lässt sich das Implantat leichter auf die Implantataufnahme aufstecken. Zudem können Beschädigungen des Implantats und Verletzungen des OP-Personals verhindert werden.

Wenn der Träger in einem Container eingesetzt ist, der mit einem Deckel verschliessbar oder verschlossen ist, ist es vorteilhaft, wenn der erste Teil der Implantataufnahme mit dem Deckel zumindest nahezu, insbesondere vollständig abschliesst. Damit kann eine zusätzliche Sicherung gegen ein Verlieren des gelagerten Implantats erfolgen.

Ebenfalls vorteilhaft ist es, wenn das elastische Element in einem Endbereich in Richtung des ersten Teiles so verformt ist, bevorzugt in Richtung der Implantataufnahme gekrümmt ist, dass das Implantat vor unbeabsichtigtem Verlieren gesichert ist. Bevorzugt hält der genannte Endbereich der Implantataufnahme das Implantat.

Bevorzugt ist das elastische Element an seinem Ende in Richtung des ersten Teils so geformt, bevorzugt von der Implantataufnahme weggebogen, dass das Implantat beim Einsetzen das elastische Element von der Implantataufnahme weg elastisch verformt. Das elastische Element wird also automatisch beim Einsetzen des Implantats verformt, ohne dass es hierzu eines weiteren manuellen Eingriffs bedarf.

Zweckmässig ist es auch, wenn der Träger nur ein einziges elastisches Element aufweist. Hierdurch entsteht eine baulich einfache Ausführungsform. Natürlich ist es auch denkbar, dass der Träger zwei oder mehr elastische Elemente mit den oben genannten Eigenschaften aufweist.

Mit weiterem Vorteil weist der Träger zusätzlich einen Stopper mit einem Anschlag für das elastische Element auf, welcher so geformt und angeordnet ist, dass eine plastische Verformung des elastischen Elements, insbesondere ein Überbiegen, verhindert wird. Beispielsweise kann der Anschlag als insbesondere gebogenes Blech oder als weitere Implantataufnahme ausgebildet sein. Zusätzlich kann der Stopper als Schutz des Trägers dienen.

In diesem Zusammenhang ist es vorteilhaft, wenn der Stopper an dem zweiten Teil der Implantataufnahme befestigt ist, und zwar bevorzugt mittels eines Gewindes. Der Vorteil eines Gewindes besteht darin, dass der Stopper gleichzeitig als Mutter zur Befestigung des Trägers an einem Boden eines Containers dienen kann. Alternativ zu einem Gewinde kann die Befestigung aber auch durch eine Rastverbindung (Einschnappen), eine Pressverbindung (Einpressen), eine Klebeverbindung (Einkleben) oder eine Schweissverbindung erfolgen. Bei dem Gewinde kann es sich beispielsweise um ein M3-Gewinde handeln. Des Weiteren kann der Stopper eine Öffnung für das elastische Element aufweisen, so dass das elastische Element durch den Stopper hindurchgeführt werden kann. Zudem ist es möglich, dass der Stopper mit dem elastischen Element einstückig ausgebildet ist. Beispielsweise können Stopper und elastisches Element durch verschiedene Bereiche eines einstückigen gebogenen Bleches gebildet sein.

Vorteilhaft ist es ausserdem, wenn der erste Befestigungsabschnitt der Implantataufnahme eine konische Form aufweist. Auf diese Weise kann ein Spiel zwischen der Implantataufnahme und einer Öffnung in einem Boden eines Containers verhindert werden. Dies kann zwar auch über enge Toleranzen realisiert werden. Eine konische Form des ersten Befestigungsabschnitts hat jedoch den Vorteil einer genaueren Positionierbarkeit.

Des Weiteren ist es von Vorteil, wenn die Implantataufnahme im zweiten Teil ein Gewinde und eine Querschnittsverdickung, bevorzugt einen Kopf, aufweist. Somit kann ein Boden der Containers zwischen der Querschnittsverdickung und dem Stopper eingeklemmt werden. Zweckmässigerweise ist dabei der konische erste Befestigungsabschnitt entlang einer Längsachse der Implantataufnahme zwischen Gewinde und Querschnittsverdickung angeordnet. Somit kann die Querschnittsverdickung unterhalb des Halteabschnitts des Containers angeordnet werden und das Gewinde oberhalb davon.

In einigen Ausführungsformen weist das elastische Element einen zweiten Befestigungsabschnitt mit einer Ausnehmung auf, durch welche die Implantataufnahme zumindest teilweise durchgeführt oder durchführbar ist, so dass das elastische Element an der Implantataufnahme anbringbar ist. Hierdurch ergibt sich eine kompakte Bauweise.

Bevorzugt ist es auch, wenn das elastische Element einen zweiten Befestigungsabschnitt aufweist, welcher zwischen den oben genannten Stopper und einer Querschnittsverdickung, bevorzugt einem Kopf, der Implantataufnahme einklemmbar ist. Diese Ausführungsform ermöglicht eine Klemmung zwischen dem Kopf und dem Stopper.

Mit weiterem Vorteil weist das elastische Element einen zweiten Befestigungsabschnitt auf, welcher zumindest einen ersten Fortsatz, bevorzugt mindestens zwei, besonderes bevorzugt genau zwei erste Fortsätze, aufweist, mit dem/denen das elastische Element am Halteabschnitt des Containers ausrichtbar ist. Die Ausrichtung durch einen derartigen Fortsatz ist baulich besonders einfach. Alternativ ist es natürlich auch denkbar, dass der Halteabschnitt Fortsätze aufweist, mit denen das elastische Element am Halteabschnitt des Containers ausrichtbar ist.

Ebenfalls bevorzugt ist es, wenn das elastische Element einen zweiten Befestigungsabschnitt aufweist, welcher zumindest einen zweiten Fortsatz, bevorzugt mindestens zwei, besonderes bevorzugt genau zwei zweite Fortsätze, aufweist, mit dem/denen der Stopper an dem elastischen Element ausrichtbar ist.

Bevorzugt erstrecken sich die ersten und/oder die zweiten Fortsätze entlang der Längsachse der Implantataufnahme. Die ersten Fortsätze und die zweiten Fortsätze können sich in entgegengesetzten Richtungen entlang der Längsachse der Implantataufnahme erstrecken. Die Fortsätze sind bevorzugt als Flügel ausgebildet, was wiederum eine einfache Bauart darstellt. Ebenfalls bevorzugt erstrecken sich die Fortsätze in der Längsachse der Implantataufnahme. Alternativ können die Fortsätze auch an dem oben genannten Stopper angeordnet sein.

Die Implantataufnahme kann beispielsweise aus rostfreiem Stahl, insbesondere aus Inox A2 bestehen und als Drehteil gefertigt werden. Das elastische Element kann beispielsweise aus einem Federstahl bestehen und als Stanzteil und/oder Biegeteil gefertigt werden. In vorteilhaften Ausführungsformen ist das elastische Element einstückig ausgebildet. Auch der Stopper kann beispielsweise aus rostfreiem Stahl, insbesondere aus Inox A2 bestehen und als Stanzteil und/oder Biegeteil gefertigt werden.

Ein zweites Beispiel der vorliegenden Offenbarung betrifft ein Set, welches einen wie oben beschriebenen Träger sowie ein chirurgisches Implantat enthält, insbesondere ein Implantat, das mit dem Träger in der oben beschriebenen Weise zusammenwirkt. Das Implantat weist eine Ausnehmung auf (beispielsweise eine Ausnehmung einer Knochenplatte), in welcher die Implantataufnahme aufnehmbar ist und/oder durch welche die Implantataufnahme durchführbar ist, um das Implantat zu halten. Durch die Kombination des Trägers mit dem Implantat ergeben sich die bereits oben erläuterten Vorteile.

Bevorzugt ist die Ausnehmung bevorzugt zumindest teilweise rund. Die Ausnehmung kann ganz oder teilweise gekrümmt oder kreisrund sein. Sie kann an zumindest einem Abschnitt die Form der Implantataufnahme aufweisen. Der Querschnitt des Implantats kann geometrisch ähnlich oder sogar kongruent zum Querschnitt der Ausnehmung sein. Diese Massnahmen erleichtern die Positionierung des Implantats an der Implantataufnahme.

In einem dritten Beispiel betrifft die vorliegende Offenbarung ein Set, das einen wie oben beschriebenen Träger und ein Sperrelement für den Träger enthält, wobei das Sperrelement einen Körper mit einer Aufnahmeöffnung aufweist, durch welche der Schaft der Implantataufnahme durchführbar ist. Das Sperrelement dient als Platzhalter und verhindert, dass eine oder mehrere Knochenplatten an einer Position eingelegt/aufgenommen werden können, an der keine Bestückung erwünscht ist.

Vorzugsweise weist der Körper eine Einkerbung für das elastische Element auf. Alternativ kann das Sperrelement zumindest teilweise elastisch ausgebildet sein. Beide Massnahmen sorgen dafür, dass das Sperrelement an der Implantataufnahme sicherbar ist.

Ein viertes Beispiel der vorliegenden Offenbarung besteht in einem Container für chirurgische Implantate, insbesondere für Knochenplatten. Der Container enthält zumindest einen Boden mit einem Halteabschnitt, wobei am Halteabschnitt, bevorzugt direkt am Halteabschnitt, ein wie oben beschriebener Träger und/oder ein wie oben beschriebenes Set befestigt ist. Die Vorteile eines solchen Containers ergeben sich aus den obigen Ausführungen zum Träger und zum Set.

Sofern der Container einen Träger gemäss dem ersten Beispiel enthält, so erstreckt sich der Schaft der Implantataufnahme des Trägers bevorzugt im Wesentlichen senkrecht zum Boden. Auf diese Weise können mehrere Träger besonders platzsparend nebeneinander angeordnet werden.

Mit weiterem Vorteil weist der Halteabschnitt zusätzlich mindestens eine Ausrichtungsausnehmung für die oben beschriebenen ersten Fortsätze des Trägers auf. Hierdurch entsteht eine Übergangspassung, die vorteilhaft ist, um die Haltekraft durch den Abstand zwischen den Kontaktflächen der Ausrichtungsausnehmung einerseits und den Kontaktflächen der Fortsätze andererseits einzustellen; denn je näher die genannten Kontaktflächen einander sind, desto präziser kann der Träger positioniert werden. Die Ausrichtungsausnehmung kann beispielsweise gestanzt oder gelasert werden. Sie kann mehrere Bereiche aufweisen, beispielsweise einen insbesondere kreisförmigen Bereich zur Aufnahme der Implantataufnahme und mindestens einen insbesondere im Wesentlichen rechteckigen Aufnahmebereich zur Aufnahme eines wie oben beschriebenen ersten Fortsatzes des elastischen Elements.

Die vorliegende Erfindung betrifft einen Träger zur Aufnahme zumindest eines chirurgischen Gegenstandes, insbesondere eines Implantats, insbesondere einer Schraube. Der Träger enthält eine Halterung mit zumindest einer Aufnahme für den zumindest einen chirurgischen Gegenstand sowie ein Sicherungselement für den zumindest einen Gegenstand. Das Sicherungselement weist mindestens eine Ausnehmung zur Entnahme des zumindest einen Gegenstandes durch die Ausnehmung auf. Das Sicherungselement ist bewegbar mit der Halterung verbunden, so dass das Sicherungselement von einer ersten Position in eine zweite Position bewegbar ist. Dabei ist in der ersten Position des Sicherungselements relativ zur Halterung der zumindest eine Gegenstand gesichert, und in der zweiten Position des Sicherungselements relativ zur Halterung ist der zumindest eine Gegenstand aus der Aufnahme durch die Ausnehmung des Sicherungselements entlang einer Entnahmerichtung entnehmbar. Je nach Position des Sicherungselements ist der Gegenstand also entnehmbar oder nicht. Dies ermöglicht eine besonders gute Sicherung, die zudem visuell direkt erkennbar ist.

Die genannte Bewegung des Sicherungselements kann beispielsweise translatorisch sein. Bevorzugt erfolgt die Bewegung in einer Ebene senkrecht zur Entnahmerichtung.

Bevorzugt ist der Träger in einem Container eingesetzt, der mit einem Deckel verschliessbar ist. In einigen Ausführungsformen steht das Sicherungselement in der zweiten Position, nicht aber auch in der ersten Position, über einen Rand des Containers hervor. Solange sich das Sicherungselement in der zweiten Position befindet, kann der Deckel daher nicht auf den Container aufgesetzt werden. Dies ist nur möglich, wenn das Sicherungselement in die erste Position überführt wird, in der es nicht über den Rand des Containers hervorsteht.

In anderen Ausführungsformen ist der Träger ebenfalls in einem Container eingesetzt, der mit einem Deckel verschliessbar ist, wobei aber das Sicherungselement beim Schliessen des Deckels derart mit dem Deckel in Wirkverbindung ist oder in Wirkverbindung bringbar ist, dass das Sicherungselement durch das Schliessen des Deckels in die erste Position gebracht wird - also in die Position, in der der chirurgische Gegenstand gegen ein Herausfallen gesichert ist.

Vorteilhafterweise weist die Halterung zwei oder drei oder mehr Aufnahmen für chirurgische Gegenstände auf, und das Sicherungselement weist zu den Aufnahmen korrespondierende Ausnehmungen auf. Dabei sind die chirurgischen Gegenstände in der ersten Position in den Aufnahmen durch das Sicherungselement sicherbar, und in der zweiten Position sind die je korrespondierenden Ausnehmungen im Sicherungselement entnehmbar. Durch entsprechende Positionierung des Sicherungselements kann somit bestimmt werden, ob die chirurgischen Gegenstände gesichert werden oder entnommen werden können.

Zweckmässig ist es, wenn die Aufnahmen entlang einer geraden Linie angeordnet sind. Hierdurch kann eine platzsparende Anordnung erreicht werden.

Mit weiterem Vorzug weist der Träger, insbesondere das Sicherungselement, ein erstes Federelement auf, das derart ausgebildet und angeordnet ist, dass das Sicherungselement in der ersten Position gehalten wird - also in der Position, in der der chirurgische Gegenstand gegen ein Herausfallen gesichert ist. Das erste Federelement kann hierzu eine Federkraft ausüben, die im Wesentlichen parallel zu der Richtung wirkt, in der das Sicherungselement von der zweiten Position in die erste Position bewegbar ist.

Vorzugsweise ist das erste Federelement in einem ersten Endbereich des Sicherungselements angeordnet. Bevorzugt ist es einstückig mit dem Sicherungselement ausgebildet, wodurch sich eine besonders einfache Bauform ergibt.

Weitere Vorteile ergeben sich, wenn der Träger eine Rastvorrichtung aufweist, welche ausgebildet ist, in einer Rastposition einzurasten und das Sicherungselement in der zweiten Position zu fixieren, sobald das Sicherungselement die zweite Position erreicht. Hierdurch wird bewirkt, dass das Sicherungselement nicht versehentlich in die erste Position zurückkehren und somit die Entnahme des Gegenstandes verhindern kann. Zweckmässigerweise ist die Rastvorrichtung lösbar, so dass das Sicherungselement wieder in die erste Position überführbar ist, in der ein noch nicht entnommener Gegenstand gegen ein Herausfallen gesichert wird.

Das Sicherungselement kann weiterhin ein Betätigungselement aufweisen, welches beim Betätigen das Sicherungselement in die erste Position bewegt. Dabei kann das Betätigungselement derart ausgebildet und angeordnet sein, dass es beim vollständigen Aufsetzen eines Deckels auf den Container vom Deckel bewegt wird, insbesondere in der Richtung, in der das Sicherungselement in die zweite Position bewegbar ist. Das Betätigungselement kann in einem gegenüber vom ersten Endbereich insbesondere einstückig angeformten zweiten Endbereich des Sicherungselements angeordnet sein.

Der zweite Endbereich kann als zweites Federelement ausgebildet sein, welches bewirkt, dass die Rastvorrichtung in der Rastposition verbleibt, wenn sich das Sicherungselement in der zweiten Position befindet. Hierzu kann das zweite Federelement eine Federkraft ausüben, die quer, insbesondere senkrecht, zu der Richtung wirkt, in der das Sicherungselement von der zweiten Position in die erste Position bewegbar ist.

In einer speziellen Ausgestaltung kann der Rastmechanismus realisiert werden, indem die Halterung des Containers über mindestens eine konturierte Aufnahmeöffnung zur Aufnahme des zweiten Endbereichs des Sicherungselements verfügt. Die Aufnahmeöffnung kann einen Durchgangsbereich aufweisen. Seitlich des Durchgangsbereichs können an der Oberseite der Halterung eine keilförmige Vertiefung mit einer geneigten oberen Gleitfläche und an der Unterseite der Halterung ein keilförmiger Vorsprung mit einer unteren Gleitfläche, einer senkrecht verlaufenden Rastfläche und einer dazwischen gebildeten Stufe vorgesehen sein. Das zweite Federelement kann dafür sorgen, dass die Rastposition eingenommen wird, wenn die weiter unten noch beschriebenen Füsse über die Stufe bewegt wurden. Durch die Kraft des ersten Federelements können die Füsse gegen die Rastfläche gedrückt werden.

Mit weiterem Vorteil entspricht die Entnahmerichtung im Wesentlichen einer Längsachse eines bestimmungsgemäss in der Ausnehmung gehaltenen chirurgischen Gegenstandes. Hierdurch können mehrere chirurgische Gegenstände platzsparend in einer senkrecht zur Längsachse verlaufenden Ebene angeordnet werden.

Ebenfalls mit Vorteil kann das Sicherungselement beschriftet sein, zum Beispiel durch eine Lasermarkierung. Die Beschriftung kann beispielsweise Informationen zu den Maßen, zur Verwendung, zur Lotnummer, zum Verfallsdatum oder zur Reihenfolge der Verwendung enthalten. Hierdurch kann beim Bestücken eines Containers leicht erkannt werden, welcher Gegenstand an welcher Position des Containers bestückt werden muss.

Ebenfalls vorteilhaft ist es, wenn das Sicherungselement zumindest zwei Füsse aufweist, die sich vom Sicherungselement in Richtung der Halterung erstrecken, wobei die Füsse elastisch ausgebildet sind und zumindest teilweise eine Gegenkontur zu einem Teil der Halterung bilden, so dass die Füsse an der Halterung einrastbar sind. Auf diese Weise kann das Sicherungselement einfach und sicher mit der Halterung verbunden werden.

Mit weiterem Vorteil sind die Füsse im eingerasteten Zustand entlang einer Kante der Halterung von der ersten Position in die zweite Position verschiebbar. Des Weiteren ist es bevorzugt, wenn die Kanten für die Füsse durch längliche Halterungsaufnahmen in der Halterung gebildet sind. Beide Massnahmen ermöglichen eine baulich einfache Realisation der Verschiebbarkeit des Sicherungselements.

Ein weiteres Ausführungsbeispiel der Erfindung ist gerichtet auf ein Set aus einem Träger gemäss der Erfindung und zumindest einem chirurgischen Gegenstand, insbesondere einem chirurgischen Implantat, insbesondere einer Schraube. Der chirurgische Gegenstand weist eine Querschnittsverdickung, bevorzugt einen Kopf, auf, welche bevorzugt an einem Rand der Aufnahme der Halterung des Trägers aufliegt. Das Sicherungselement des Trägers ist in seiner ersten Position derart angeordnet, dass die Querschnittsverdickung blockiert ist und nicht entlang der Entnahmerichtung entnehmbar ist. Hierdurch wird eine kontrollierte Entnahme des chirurgischen Gegenstandes ermöglicht.

Ein weiteres Ausführungsbeispiel der Erfindung betrifft einen Container für chirurgische Implantate, insbesondere Schrauben, wobei der Container zumindest einen, bevorzugt mehrere, Träger gemäss dem fünften Aspekt der Erfindung und/oder Sets gemäss dem sechsten Aspekt der Erfindung enthält. Die Vorteile ergeben sich aus den obigen Ausführungen.

Ein weiteres Beispiel der Offenbarung betrifft ebenfalls einen Träger zur Aufnahme eines chirurgischen Implantats mit einer Ausnehmung, insbesondere einer Knochenplatte mit einer Ausnehmung. Der Träger enthält einen Schaft zur Aufnahme des chirurgischen Implantats an einer Ausnehmung des Implantats, wobei der Schaft an einem ersten Ende Mittel zur Befestigung an einem Container aufweist. Weiterhin enthält der Träger ein Riegelelement, welches schwenkbar an einem dem ersten Ende des Schafts gegenüberliegenden zweiten Ende des Schaft angeordnet ist. Das Riegelelement kann in eine Aufnahmeposition und in eine Verriegelungsposition geschwenkt werden. In der Aufnahmeposition kann die Knochenplatte mit der Ausnehmung über das Riegelelement auf den Schaft bewegt werden. Durch Schwenken des Riegelelements in die Verriegelungsposition verhindert das Riegelelement die Entnahme der Knochenplatte.

Nachfolgend werden die Erfindung und ihre Vorteile anhand von zahlreichen Ausführungsbeispielen und Zeichnungen näher erläutert.

Bei den in den Figuren 1-14 und 25-27b gezeigten Beispielen handelt es sich nicht um Ausführungsbeispielen der Erfindung. Sie dienen lediglich dem Verständnis der Erfindung.

Dabei zeigen
- Figur 1:: einen ersten beispielhaften Träger zur Aufnahme einer Knochenplatte in einer Seitenansicht;
- Figur 2:: eine Implantataufnahme des ersten beispielhaften Trägersaus Figur 1 in einer Seitenansicht;
- Figur 3:: eine Feder des ersten beispielhaften Trägers aus Figur 1 in einer perspektivischen Ansicht;
- Figur 4:: einen Stopper des ersten beispielhaften Trägers aus Figur 1 in einer perspektivischen Ansicht;
- Figur 5:: den an einem Halteabschnitt eines Containers befestigten ersten beispielhaften Träger in einer perspektivischen Ansicht;
- Figur 6:: ein am Halteabschnitt des Containers befestigtes beispielhaftes Set mit dem ersten beispielhaften
- Figur 7a:: Träger und einer Knochenplatte in einer seitlichen Ansicht; einen Halteabschnitt eines Containers in einer Draufsicht;
- Figur 7b:: den Halteabschnitt des Containers gemäss Figur 7a mit einem daran befestigten beispielhaften Set aus dem ersten beispielhaften Träger und einer Knochenplatte;
- Figur 8:: einen ersten beispielhaften Container mit mehreren solcher Sets;
- Figur 9:: ein Sperrelement für den ersten beispielhaften Träger gemäss den Figuren 1 bis 4 in einer perspektivischen Ansicht;
- Figur 10:: das Sperrelement gemäss Figur 9 zusammen mit dem Träger gemäss den Figuren 1 bis 4 in einer Seitenansicht;
- Figur 11a:: einen zweiten beispielhaften Träger zur Aufnahme einer Knochenplatte in einer perspektivischen Ansicht;
- Figur 11b:: den zweiten beispielhaften Träger in einer Seitenansicht;
- Figur 12:: eine Feder des zweiten beispielhaften Trägers in einer perspektivischen Ansicht;
- Figur 13:: den zweiten beispielhaften Träger mit zwei Knochenplatten in einer perspektivischen Ansicht;
- Figur 14a:: einen dritten beispielhaften Träger zur Aufnahme einer oder zweier Knochenplatten in einer perspektivischen Ansicht;
- Figur 14b:: den dritten beispielhaften Träger in einer Seitenansicht;
- Figur 15:: ein Sicherungselement eines erfindungsgemässen Trägers für Schrauben in einer perspektivischen Ansicht;
- Figur 16a:: einen Ausschnitt eines erfindungsgemässen Containers, jedoch ohne Sicherungselement, in einer perspektivischen Ansicht;
- Figur 16b:: eine Detailansicht einer konturierten Aufnahmeöffnung des Containers;
- Figur 17a:: einen Ausschnitt des erfindungsgemässen Containers mit Sicherungselement in einer ersten Position, in einer perspektivischen Ansicht;
- Figur 17b:: einen Ausschnitt des erfindungsgemässen Containers mit Sicherungselement in einer zweiten Position, in einer perspektivischen Ansicht;
- Figur 18a:: eine Seitenansicht des erfindungsgemässen Containers in der zweiten Position;
- Figur 18b:: eine Seitenansicht des erfindungsgemässen Containers in der ersten Position;
- Figur 19:: den erfindungsgemässen Container in einer Draufsicht;
- Figur 20:: den erfindungsgemässen Container mit Deckel in einer perspektivischen Ansicht;
- Figur 21:: einen Ausschnitt eines zweiten erfindungsgemässen Containers mit Sicherungselement in einer ersten Position, in einer perspektivischen Ansicht;
- Figur 22:: einen Ausschnitt des zweiten erfindungsgemässen Containers mit Sicherungselement in einer zweiten Position, in einer perspektivischen Ansicht;
- Figur 23:: den zweiten erfindungsgemässen Container in einer Draufsicht;
- Figur 24:: den zweiten erfindungsgemässen Container mit Deckel in einer perspektivischen Ansicht;
- Figur 25:: ein weiteres Beispiel eines Trägers zur Aufnahme einer Knochenplatte;
- Figur 26:: eine Feder eines weiteren Trägers in drei Ansichten;
- Figur 27a:: ein weiteres Beispiel eines an einem Boden eines Containers befestigten Trägers mit einem Riegelelement in einer Aufnahmeposition für eine Knochenplatte;
- Figur 27b:: den Träger gemäss Figur 27a mit dem Riegelelement in der Verriegelungsposition.

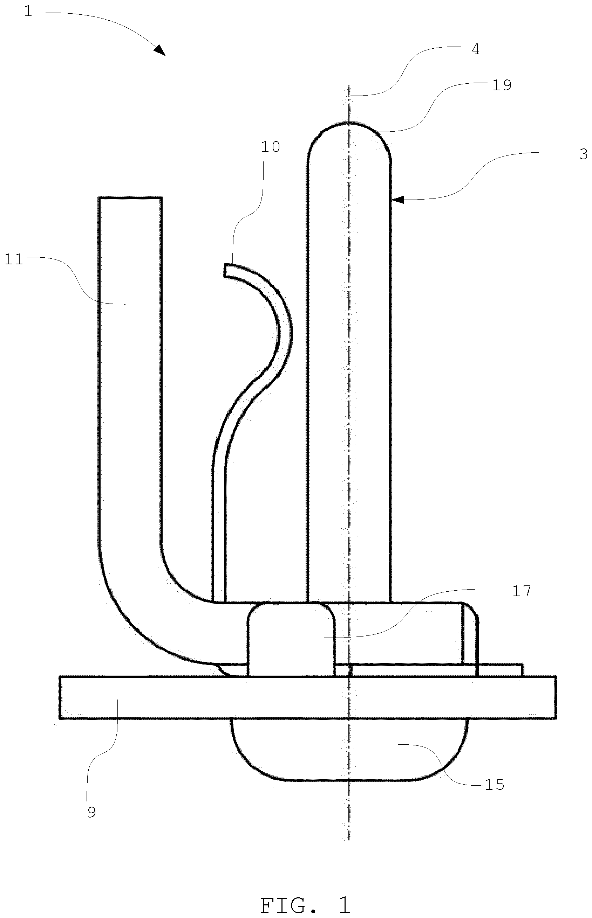

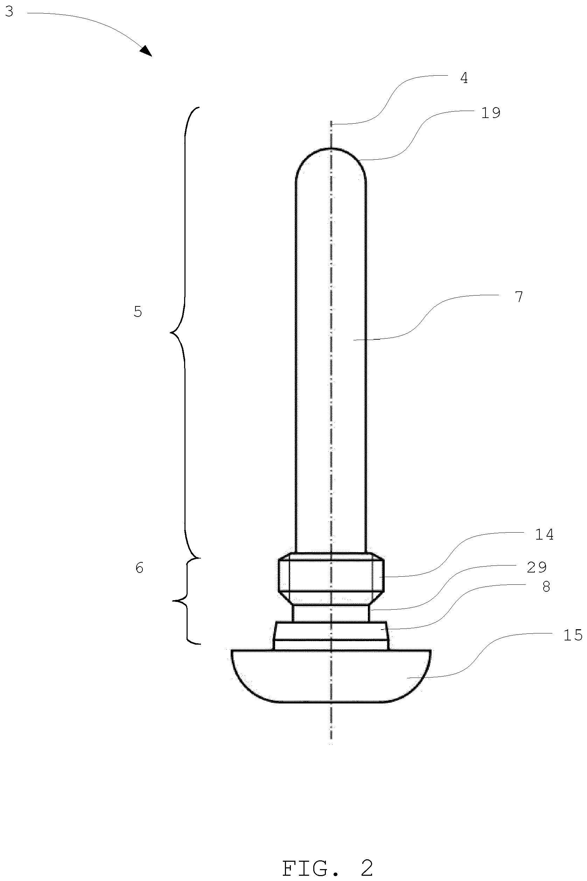

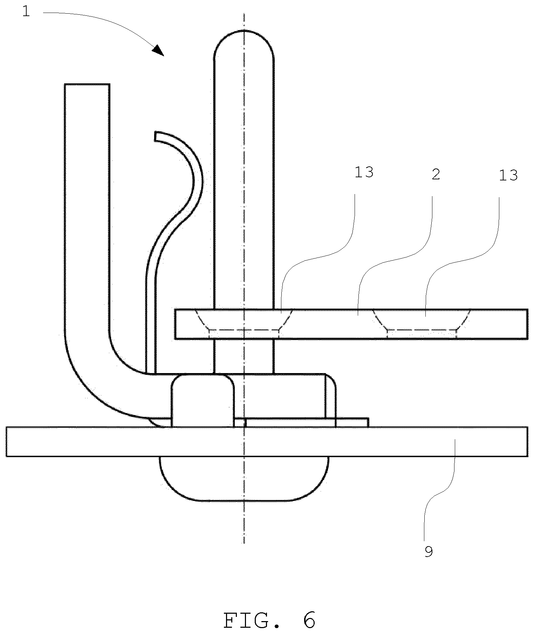

Figur 1 zeigt einen ersten beispielhaften Träger 1, der zur Aufnahme eines Implantats in Form einer hier nicht dargestellten Knochenplatte geeignet ist (siehe dazu Figur 6). Der Träger 1 enthält eine Implantataufnahme 3, ein elastisches Element in Form einer Feder 10 sowie einen Stopper 11, die in den Figuren 2 bis 4 im Detail dargestellt sind.

Gemäss Figur 2 ist die Implantataufnahme 3 im Wesentlichen länglich entlang einer Längsachse 4 ausgebildet und weist einen ersten Teil 5 und einen zweiten Teil 6 auf. Der erste Teil 5 enthält einen Schaft 7 zur Aufnahme eines oder mehrerer Implantate an jeweiligen Ausnehmungen des Implantats/der Implantate. Auf der Seite des ersten Teils 5 verfügt die Implantataufnahme 3 über ein gerundetes Ende 19 zur Aufnahme des Implantats. Der zweite Teil 6 enthält ein Gewinde 14, beispielsweise ein M3-Gewinde, einen ersten, konisch ausgebildeten Befestigungsabschnitt 8 zur Befestigung an einem hier nicht dargestellten Halteabschnitt eines Containers (siehe dazu Figur 6) sowie eine Querschnittsverdickung in Form eines Kopfes 15. Zwischen dem Gewinde 14 und dem Befestigungsabschnitt 8 befindet sich ein Gewindefreistich 29. Der erste Befestigungsabschnitt 8 ist entlang der Längsachse 4 der Implantataufnahme 3 zwischen dem Gewinde 14 und der Querschnittsverdickung 15 angeordnet. Wie Figur 1 zeigt, ist der Stopper 11 am zweiten Teil 6 der Implantataufnahme 3 befestigt, und zwar mittels des Gewindes 14. Wie sich ebenfalls aus Figur 1 ergibt, dass sich die Implantataufnahme 3 entlang der Längsachse 4 in Richtung des ersten Teils 5 über die Feder 10 hinaus erstreckt und dass der zweite Befestigungsabschnitt 12 der Feder 10 zwischen dem Stopper 11 und dem Kopf 15 der Implantataufnahme 3 eingeklemmt ist.

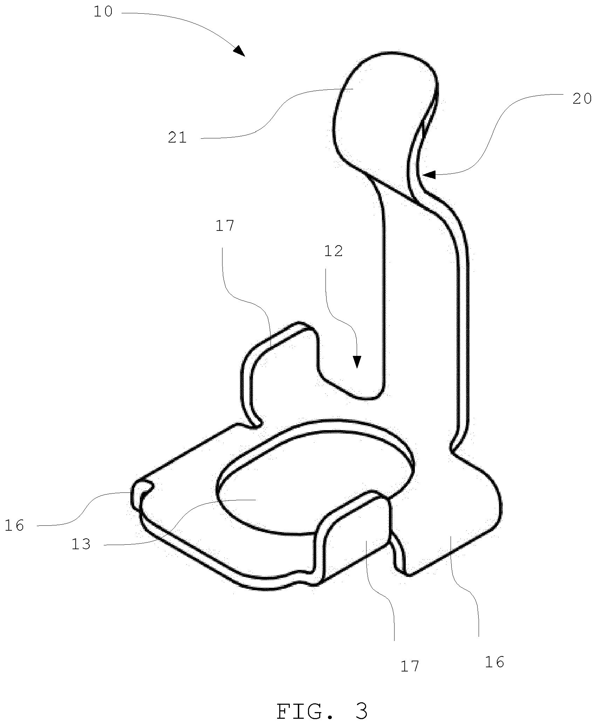

Die in Figur 3 dargestellte Feder 10 erstreckt sich entlang der Längsachse 4 der Implantataufnahme 3 (siehe Figur 1). Die Feder 10 ist senkrecht zu dieser Richtung elastisch und so angeordnet, dass sie bei Entnahme des Implantats elastisch verformt wird (siehe Figur 6). Dies sorgt für eine Kraftkomponente senkrecht zur Längsachse 4, wodurch ein an der Implantataufnahme 3 gehaltenes Implantat gesichert werden kann. Die Feder 10 dient somit als Verlustsicherung.

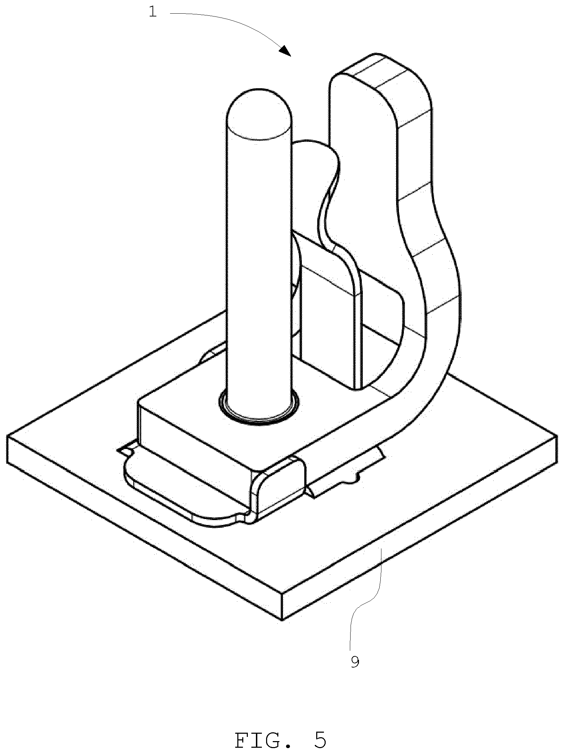

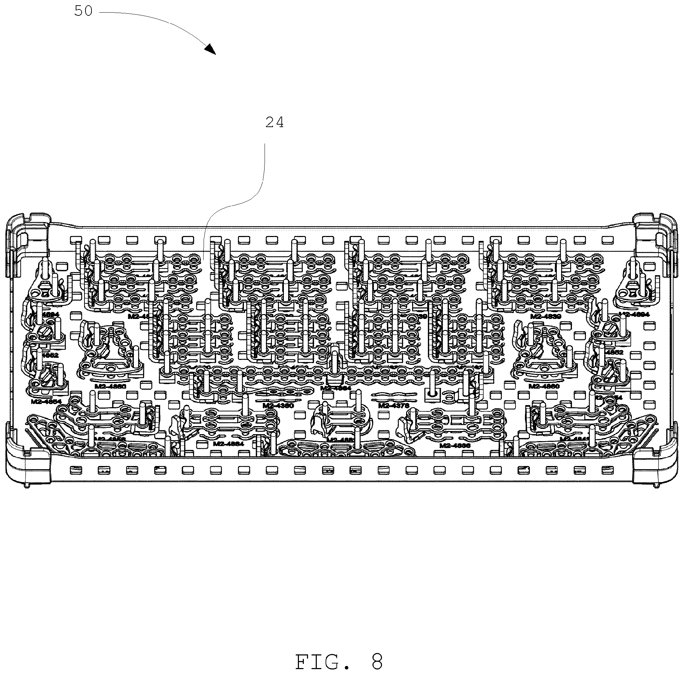

In Figur 3 ist weiterhin erkennbar, dass die Feder 10 einen zweiten Befestigungsabschnitt 12 mit einer Ausnehmung 13 aufweist, durch welche die Implantataufnahme 3 durchführbar ist, wodurch die Feder 10 an der Implantataufnahme 3 anbringbar ist (siehe Figur 1). Der zweite Befestigungsabschnitt 12 verfügt über zwei erste flügelförmige Fortsätze 16. Mit den ersten Fortsätzen 16 ist die Feder 10 an einem hier nicht dargestellten Halteabschnitt eines Containers ausrichtbar (siehe dazu Figur 8). Überdies weist der zweite Befestigungsabschnitt 12 zwei zweite Fortsätze 17 auf, mit denen der Stopper 11 an der Feder 10 ausrichtbar ist (siehe dazu Figur 5). Die ersten Fortsätze 16 und die zweiten Fortsätze 17 erstrecken sich in entgegengesetzten Richtungen bezüglich der Längsachse 4 der Implantataufnahme 3. In einem Endbereich 20 ist die Feder 10 in Richtung des ersten Teils 5 der Implantataufnahme 3 derart gekrümmt, dass das Implantat vor unbeabsichtigtem Verlieren gesichert ist (siehe dazu Figuren 1 und 6). An einem Ende 21 ist die Feder 10 in Richtung des ersten Teils 5 der Implantataufnahme 3 derart weggebogen, dass ein Implantat 2 beim Einsetzten die Feder 10 elastisch verformt.

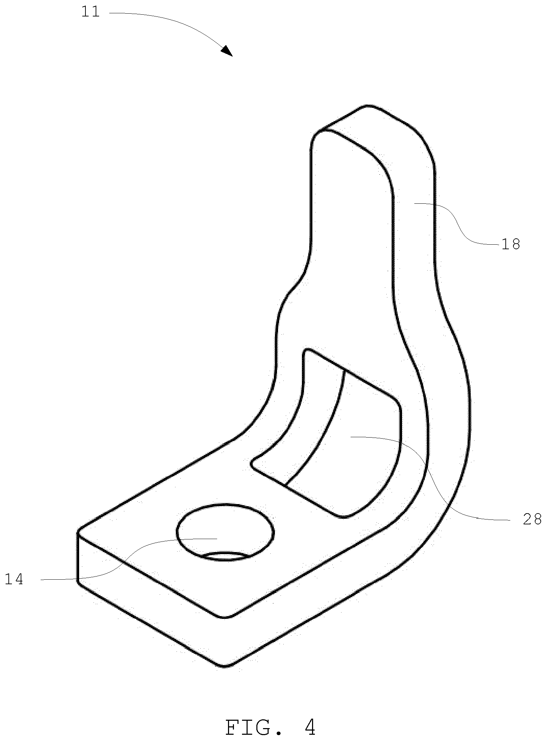

Der in Figur 4 einzeln dargestellte Stopper 11 verfügt über einen Anschlag 18 für die Feder 10. Der Anschlag 18 ist so geformt und angeordnet, dass eine plastische Verformung der Feder 10, insbesondere ein Überbiegen, verhindert wird. Ausserdem verfügt der Stopper 11 über eine Öffnung 28, durch die die Feder 10 durchgeführt werden kann.

Die Figur 5 zeigt den an einem Halteabschnitt 9 eines Containers befestigten Träger 1 in einer perspektivischen Ansicht.

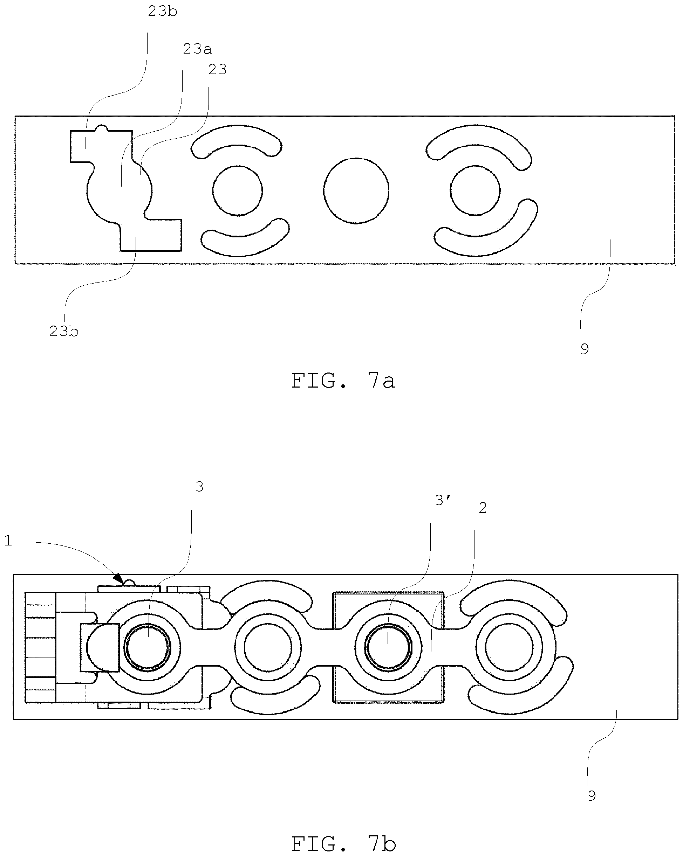

In Figur 6 ist ein beispielhaftes Set mit dem ersten erfindungsgemässen Träger 1 und einer Knochenplatte 2 dargestellt. Dieses Set ist am Halteabschnitt 9 des Containers befestigt. Die Figuren 7a und 7b zeigen einen Ausschnitt eines Halteabschnitts 9 eines hier nicht gesamthaft dargestellten Containers, und zwar in Figur 7a ohne Knochenplatte und in Figur 7b mit einer Knochenplatte 2. Der Halteabschnitt 9 verfügt über eine Ausrichtungsausnehmung 23 für die ersten Fortsätze 16 der Feder 10. Die Ausrichtungsausnehmung 23 ist zusammengesetzt aus einem mittleren, kreisförmigen Bereich 23a und zwei damit verbundenen, einander gegenüberliegenden, im Wesentlichen rechteckigen Aufnahmebereichen 23b. Im kreisförmigen Bereich 23a wird die Implantataufnahme 3 gehalten und mittels des in Figur 2 dargestellten konischen Befestigungsabschnitts 8 zentriert. In den Aufnahmebereichen 23b werden die ersten Fortsätze 16 der Feder 10 gehalten. Die Knochenplatte 2 wird zudem durch eine weitere Implantataufnahme 3' gehalten, die identisch zur Implantataufnahme 3 ist und auf gleiche Weise am Halteabschnitt 9 befestigt ist wie in Figur 6 dargestellt.

In Figur 8 ist ein erster beispielhafter Container 50 mit mehreren solcher Sets dargestellt. Wie der Figur zu entnehmen ist, hält jeder Träger zusammen mit einer jeweiligen weiteren Implantataufnahme eine Knochenplatte.

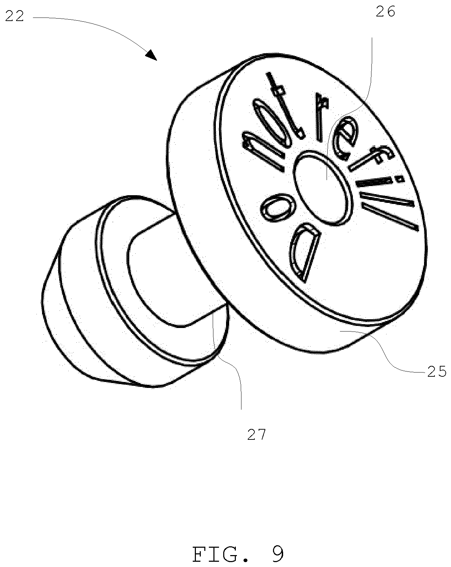

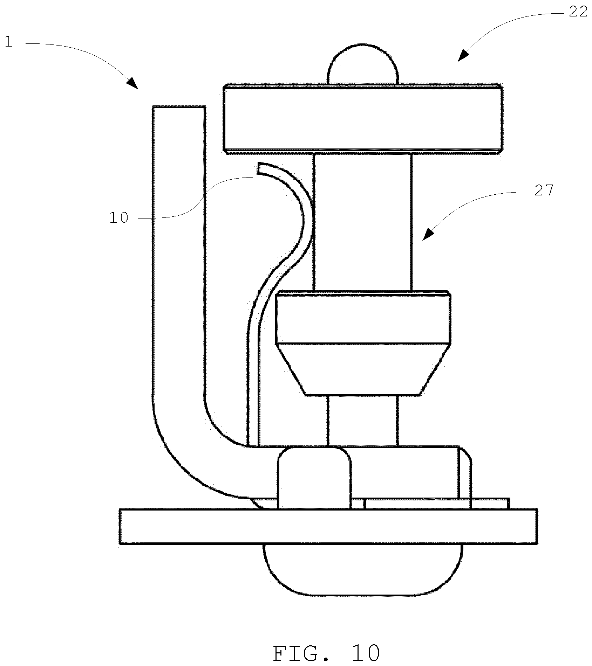

Figur 9 zeigt ein elastisches Sperrelement 22 für den Träger 1 gemäss den Figuren 1 bis 4. Das Sperrelement 22 weist einen Körper 25 mit einer Aufnahmeöffnung 26 auf, durch welche der Schaft 7 der Implantataufnahme 3 durchführbar ist (siehe dazu Figur 10). Der Körper 25 weist weiterhin eine Einkerbung 27 auf, in die die Feder 10 eingreift. Auf diese Weise kann das Sperrelement 22 an der Implantataufnahme 3 gesichert werden (siehe Figur 10). Das Sperrelement 22 verhindert das Aufsetzen von Knochenplatten auf die Implantataufnahme 3.

Die bereits erwähnte Figur 6 zeigt ein Set, das aus einem Träger 1 und einer Knochenplatte 2 mit zwei Ausnehmungen 13 besteht. Die Implantataufnahme 3 des Trägers 1 ist in einem kreisförmigen Bereich 23a des Halteabschnitts 9 aufgenommen und durch eine der Ausnehmungen 13 geführt. Hierdurch wird die Knochenplatte 2 gehalten.

Die ebenfalls bereits erwähnte Figur 8 zeigt einen ersten erfindungsgemässen Container 50 für Knochenplatten 2. Der Container 50 weist einen Boden 24 mit einem Halteabschnitt auf, wobei direkt am Halteabschnitt (also ohne irgendwelche dazwischen angeordnete Bauteile) ein Set gemäss Figur 6 befestigt ist. Die Schäfte 7 erstrecken sich senkrecht zum Boden 24.

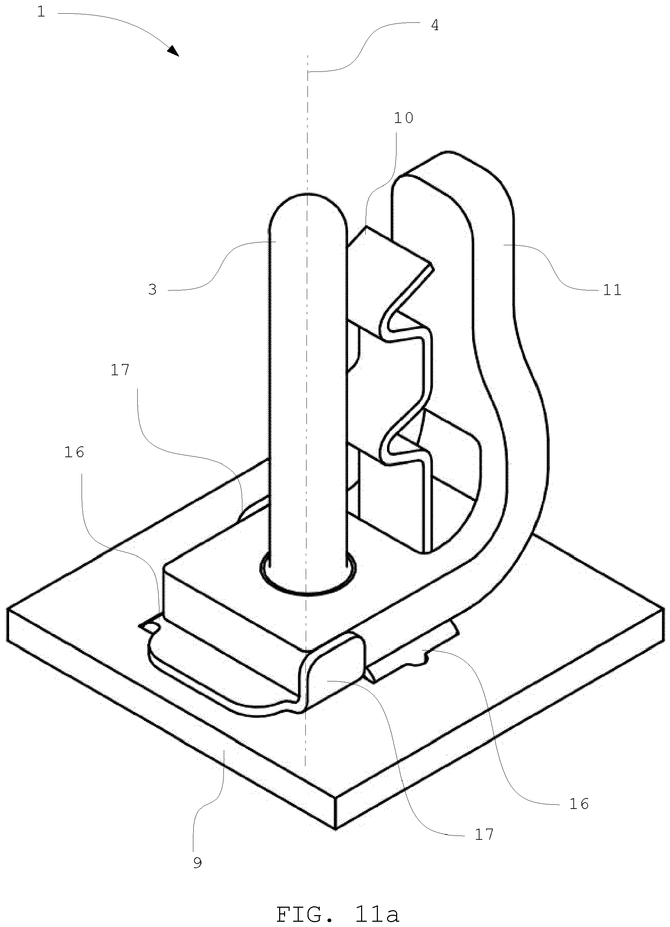

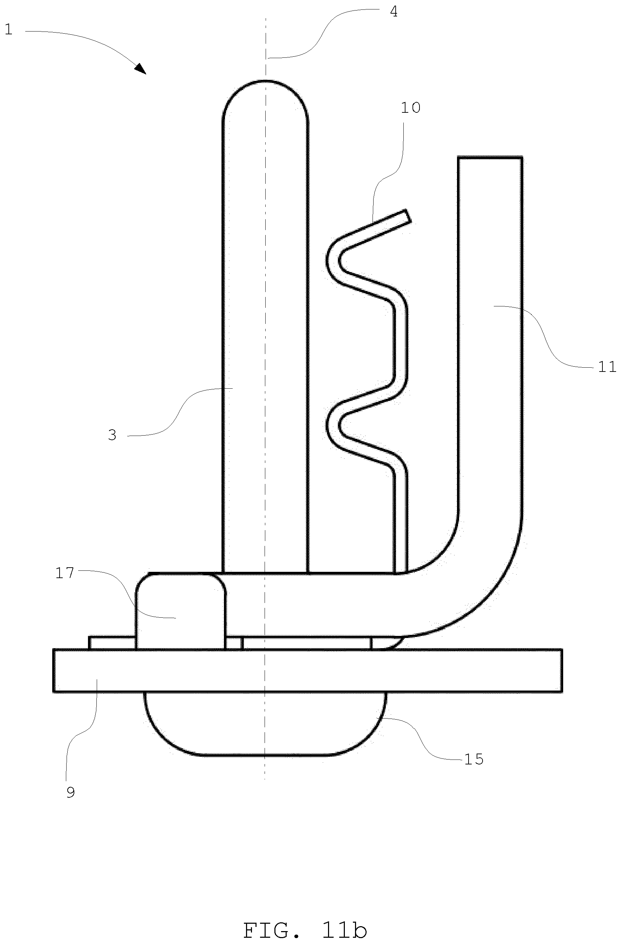

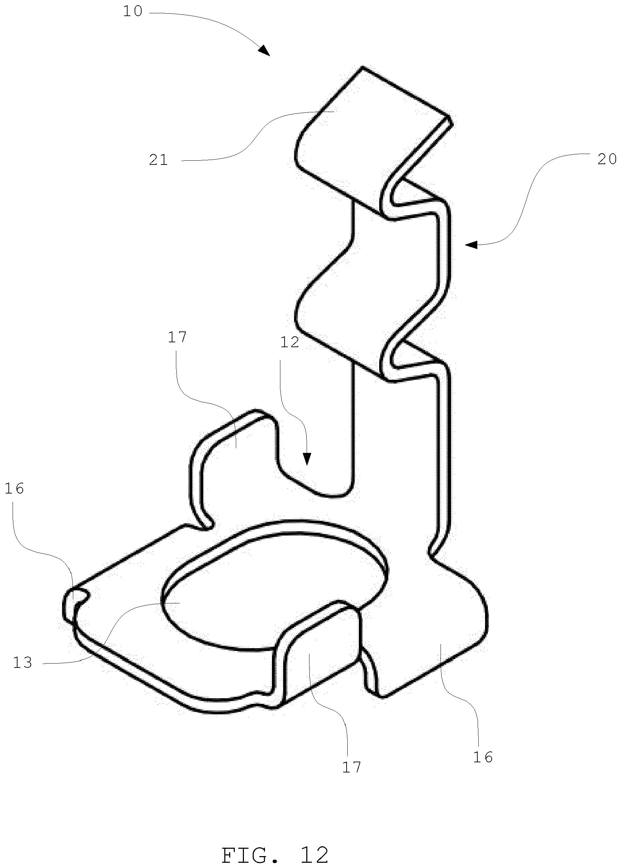

Die Figuren 11a und 11b zeigen einen zweiten beispielhaften Träger 1 in einer perspektivischen und einer Seitenansicht. Dieser Träger 1 unterscheidet sich von dem in den Figuren 1 bis 4 dargestellten im Wesentlichen in der Feder 10, die in Figur 12 separat dargestellt ist. In ihrem Endbereich 20 ist die Feder 10 nicht nur an einer, sondern an zwei Stellen in Richtung der Implantataufnahme 3 gekrümmt. Hierdurch können zwei Implantate in einem Abstand voneinander gehalten werden, wodurch die Implantate auch besser gereinigt und insbesondere sterilisiert werden können.

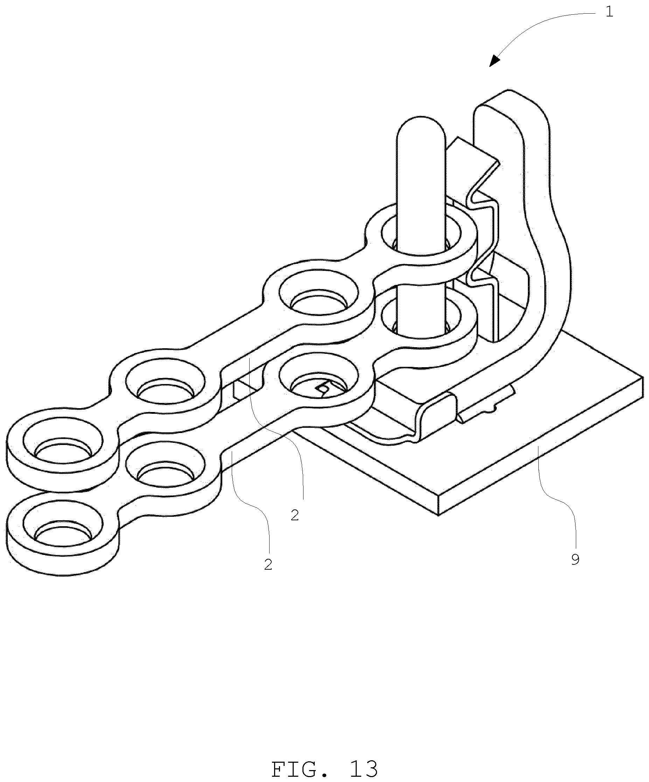

Der zweite beispielhafte Träger 1 gemäss den Figuren 11a und 11b ist in Figur 13 zusammen mit zwei daran gehaltenen Knochenplatten 2 gezeigt. Der Träger 1 ist auch hier an einem Halteabschnitt 9 eines Containers befestigt.

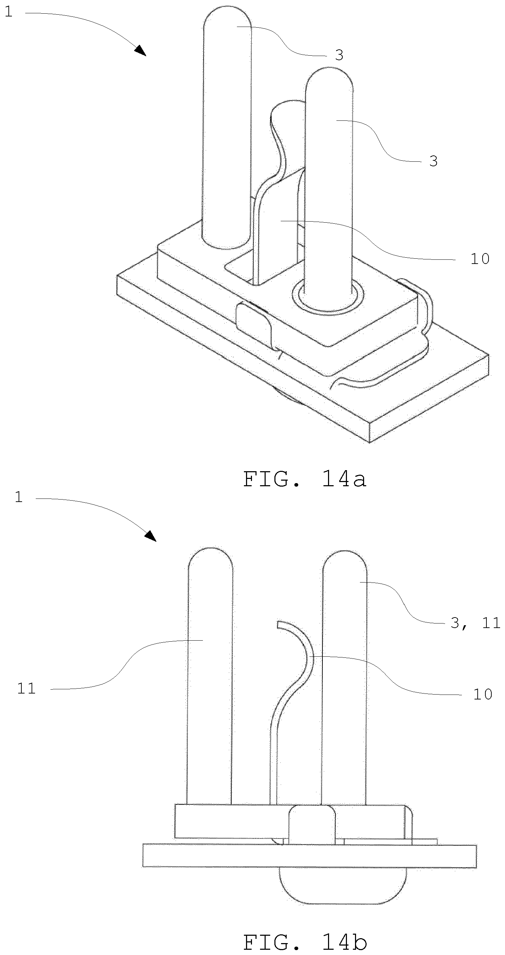

Eine weitere Ausführungsform ist in den Figuren 14a und 14b gezeigt. Dieser Träger 1 enthält zwei Implantataufnahmen 3 und eine Feder 10. Wie der Figur 14b zu entnehmen ist, fungiert die dort rechts dargestellte Implantataufnahme 3 zugleich als Stopper 11 für die Feder 10. Dieser Träger 1 kann eine einzige Knochenplatte aufnehmen, die von einer oder beiden Implantataufnahmen 3 gehalten wird, oder zwei Knochenplatten, die an jeweils einer der beiden Implantataufnahmen 3 gehalten werden.

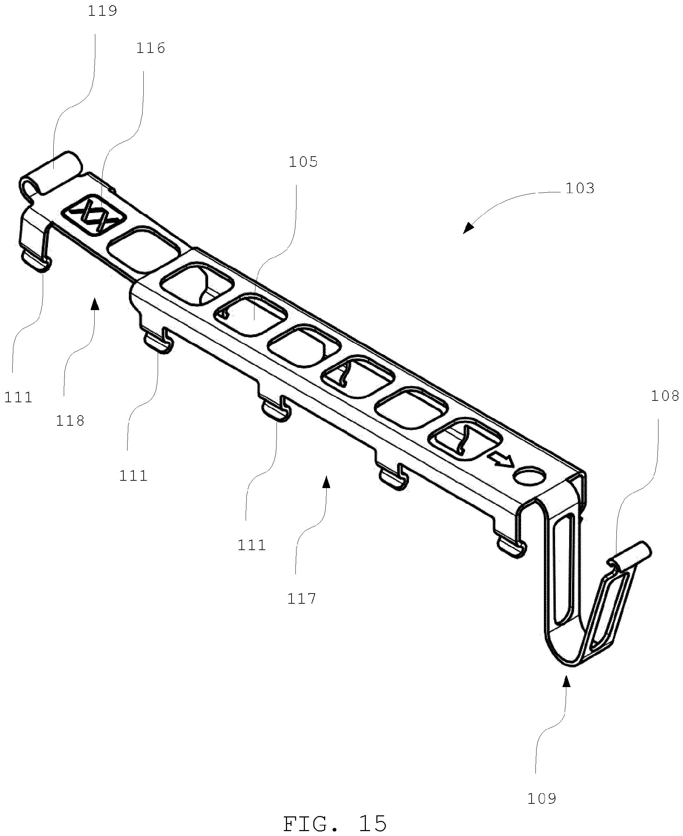

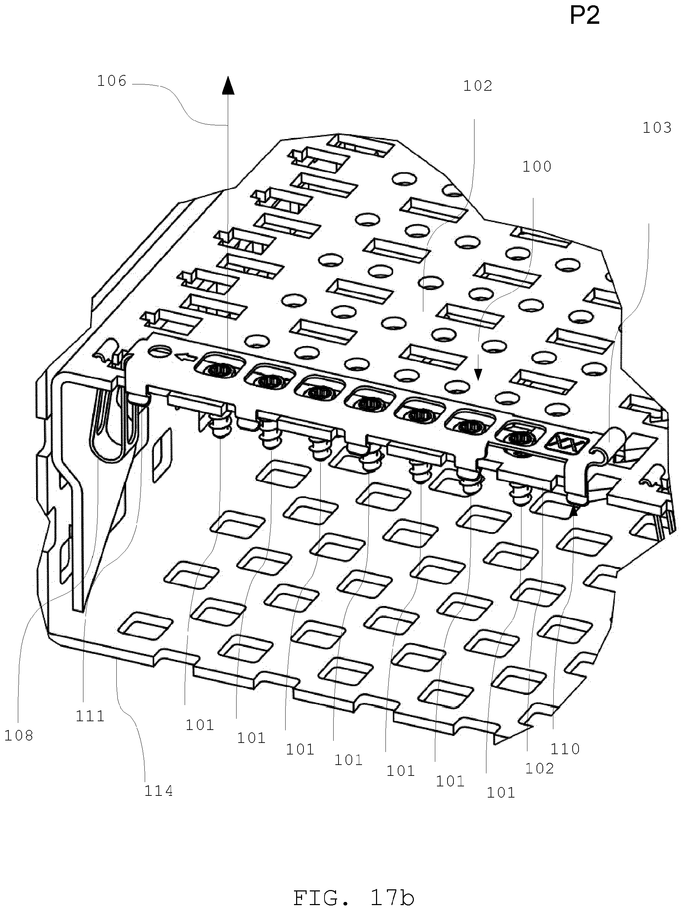

Figur 15 zeigt ein erfindungsgemäßes Sicherungselement 103 eines weiteren erfindungsgemässen Trägers, der jedoch zur Aufnahme einer oder mehrerer Schrauben vorgesehen ist. Das Sicherungselement 103 enthält einen Mittelbereich 117 mit mehreren Ausnehmungen 105, durch die eine Schraube entnommen werden kann (siehe dazu Figur 17b), sowie mehrere elastisch ausgebildete Füsse 111. In einem ersten Endbereich 109 weist das Sicherungselement 103 ein einstückig angeformtes erstes Federelement 108 auf. Gegenüber vom ersten Endbereich 109 verfügt das Sicherungselement 103 über einen ebenfalls einstückig angeformten zweiten Endbereich 118, der ein zweites Federelement bildet und ebenfalls elastisch ausgebildete Füsse 111 aufweist. Im zweiten Endbereich 118 ist das Sicherungselement 103 durch eine Lasermarkierung 116 beschriftet. Im äussersten Ende des zweiten Endbereichs 118 ist ein sich von den Füssen 111 weg, also nach oben erstreckendes Betätigungselement 119 angeformt.

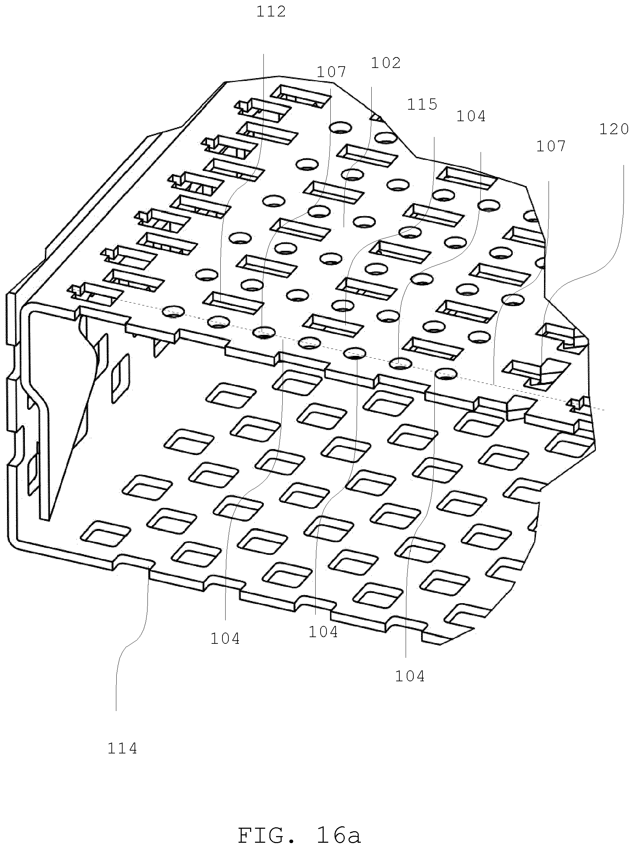

Figur 16a zeigt einen Ausschnitt eines erfindungsgemässen Containers 114 mit einer Halterung 102. Die Halterung 102 verfügt über mehrere Aufnahmen 104 für jeweils eine Schraube. Die Aufnahmen 104 sind entlang einer geraden Linie 107 angeordnet. Die Ausnehmungen 105 des Sicherungselements 103 korrespondieren zu den Aufnahmen 104 der Halterung 102. Weiterhin verfügt die Halterung 102 über längliche Halterungsausnehmungen 112, die entlang einer zur Linie 107 parallelen Linie angeordnet sind. Zudem weist die Halterung 102 mehrere konturierte Aufnahmeöffnungen 120 auf.

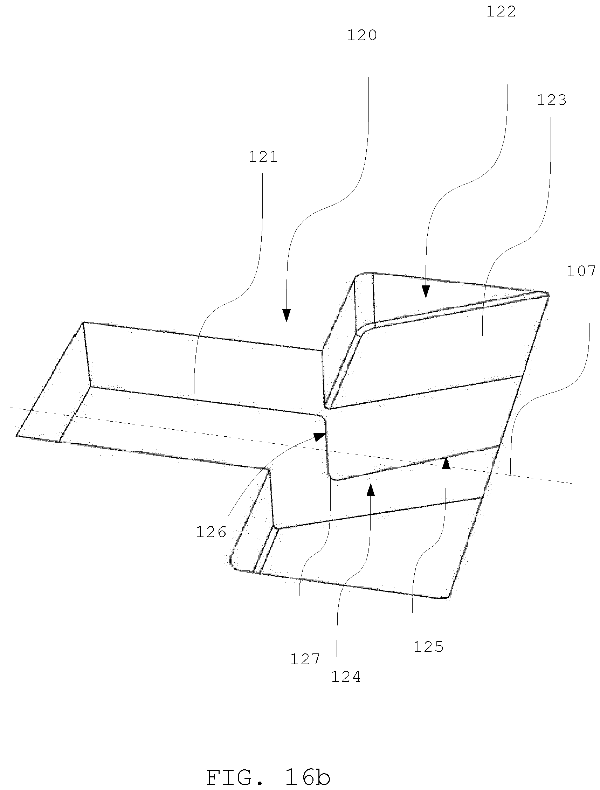

Figur 16b enthält eine Detailansicht einer der konturierten Aufnahmeöffnungen 120. Die Aufnahmeöffnung 120 weist einen rechteckigen Durchgangsbereich 121 mit einer längeren Seite und einer kürzeren Seite auf, wobei sich die längere Seite parallel zur Linie 107 erstreckt. Beidseits des Durchgangsbereichs 121 sind an der Oberseite der Halterung 102 eine keilförmige Vertiefung 122 mit einer geneigten oberen Gleitfläche 123 und an der Unterseite der Halterung 102 ein keilförmiger Vorsprung 124 mit einer unteren Gleitfläche 125, einer senkrecht verlaufenden Rastfläche 126 und einer dazwischen gebildeten Stufe 127 vorgesehen.

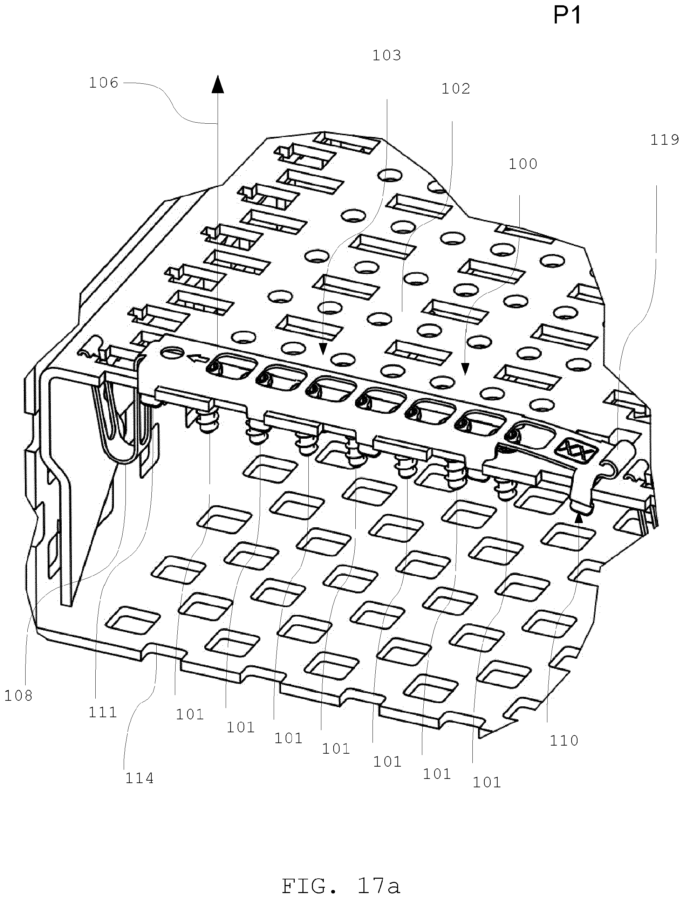

Die Figuren 17a und 17b zeigen den Container 114 mit der Halterung 102 und einem Sicherungselement 103. In jeder der Aufnahmen 104 ist eine Schraube 101 eingesetzt. Das erste Federelement 108 ist in einer Öffnung der Halterung 102 eingesetzt und die Füsse 111 des zweiten Endbereichs 118 in zwei benachbarten konturierten Aufnahmeöffnungen 120. Die Füsse 111 des Mittelbereichs 117 erstrecken sich in Richtung der Halterung 102 und sind in den Halterungsausnehmungen 112 eingerastet. Die Längsausdehnung der Füsse 111 des Mittelbereichs 117 in Richtung der Linie 107 ist geringer als die Längsausdehnung der Halterungsausnehmungen 112 in dieser Richtung. Dies hat den Effekt, dass das Sicherungselement 103 bewegbar mit der Halterung 102 verbunden ist. Auf diese Weise kann es von einer in Figur 17a dargestellten ersten Position P1 in eine in Figur17b dargestellte Position P2 verschoben, also translatorisch bewegt werden. Dies wird dadurch ermöglicht, dass die Füsse 111 im eingerasteten Zustand entlang einer Kante 115 der Halterung verschiebbar sind. Die Kanten 115 werden durch die länglichen Halterungsausnehmungen 112 der Halterung 102 gebildet.

In der in Figur 17a gezeigten ersten Position P1 sind die Schrauben 101 durch das Sicherungselement 103 gesichert. Denn da die Ausnehmungen 105 des Sicherungselements und die Aufnahmen 104 der Halterung 102 nicht miteinander fluchten, können die Schrauben 101 nicht entlang einer Entnahmerichtung 106, die mit der Längsachse der Schraube 101 übereinstimmt, entnommen werden. Dies wird lediglich in der in Figur 17b dargestellten zweiten Position P2 möglich. In dieser zweiten Position P2 ist die Schraube 101 aus der Aufnahme 104 durch die Ausnehmung 105 entlang der Entnahmerichtung 106 entnehmbar.

Das erste Federelement 108 ist derart ausgebildet und angeordnet, dass das Sicherungselement 103 in der ersten Position P1 gehalten wird. Die Figuren 17a und 17b lassen ebenfalls eine lösbare Rastvorrichtung 110 erkennen, die einrasten kann und somit das Sicherungselement 103 in der zweiten Position P2 fixieren kann, sobald das Sicherungselement 103 die zweite Position P2 erreicht.

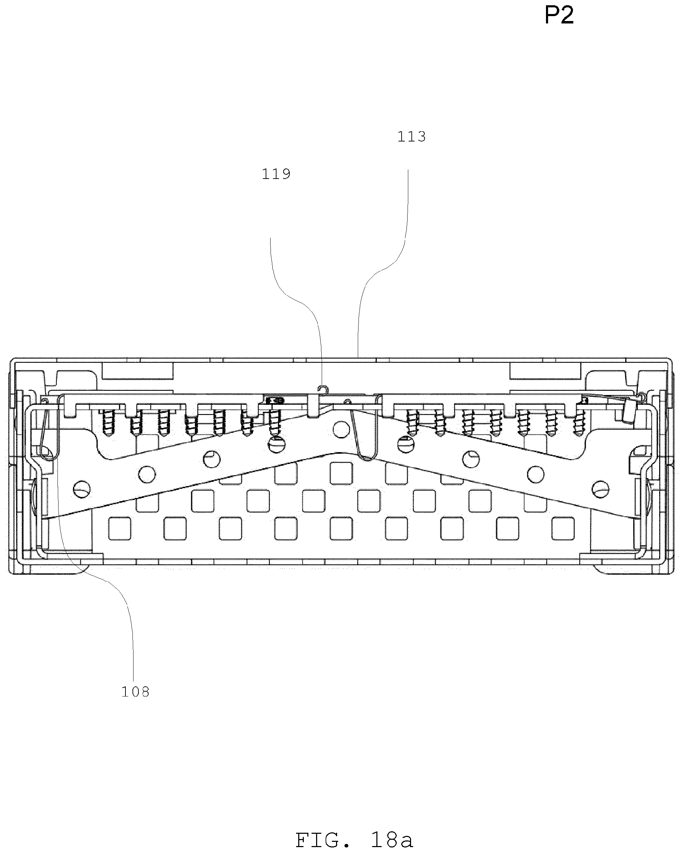

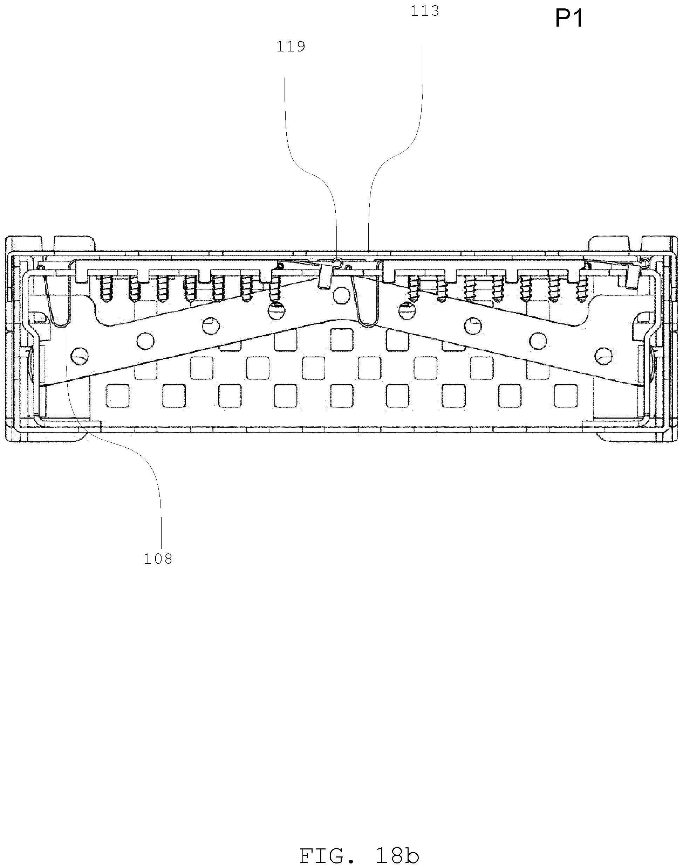

Die Figuren 18a und 18b zeigen in seitlichen Ansichten im Detail, wie die Bewegung des Sicherungselements 103 durch das Zusammenwirken mit einem Deckel 113 des Containers 50 erfolgt. In der in Figur 18a dargestellten zweiten Position P2 werden die Füsse 111 des ersten Endbereichs 118 durch das erste Federelement 108 an die Rastflächen 126 gedrückt. Der Deckel 113 ist in dieser zweiten Position P2 noch nicht vollständig aufgesetzt, so dass noch kein Kontakt mit dem Betätigungselement 119 besteht.

Dieser Kontakt entsteht erst beim vollständigen Aufsetzen des Deckels 113, wie in Figur 18b gezeigt. Der Deckel 113 drückt das Betätigungselement 119 herab. Hierdurch werden die Füsse 111 des zweiten Endbereichs 118 über die Stufe 127 bewegt, so dass sich das Sicherungselement 103 durch die vom ersten Federelement 108 erzeugte Federkraft von der zweiten Position P2 in die erste Position P1 bewegt. Dabei gleiten die Füsse 111 des zweiten Endbereichs 118 an den Gleitflächen 125 der konturierten Aufnahmeöffnungen 120 entlang.

Nach Abheben des Deckels 113 verbleibt das Sicherungselement 103 aufgrund der durch das erste Federelement 108 bewirkten Kraft in der ersten Position P1. Durch Ausübung einer parallel zur Linie 107 gerichteten Kraft auf das Betätigungselement 119 können die Füsse 111 entlang den Gleitflächen 123, 125 bis über die Stufe 127 bewegt werden, wodurch die zweite Position P2 erreicht wird und sich die Füsse 111 des zweiten Endbereichs 118 aufgrund der federnden Wirkung des ersten Endbereichs 118 nach oben und damit zurück in die Rastposition bewegen.

Die Überführung des Sicherungselements 103 von der zweiten Position P2 in die erste Position P1 kann natürlich auch manuell, also ohne Einwirkung des Deckels 113 erfolgen.

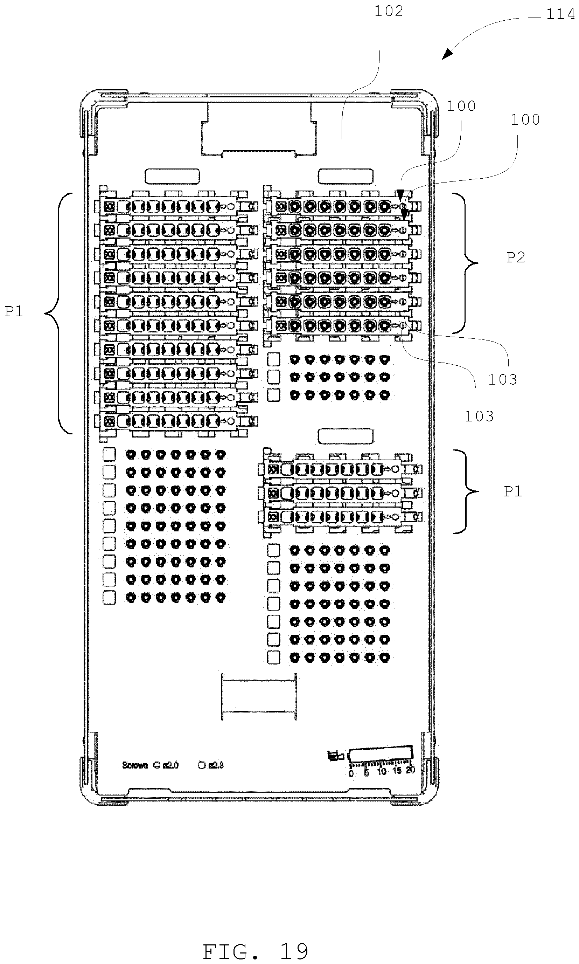

Figur 19 zeigt einen ersten erfindungsgemässen Container 114 mit einer Halterung 102 und mehreren Trägern 100, die jeweils ein Sicherungselement 103 aufweisen. Dabei befinden sich einige der Sicherungselement 103 in der ersten Position P1 und die verbleibenden in der zweiten Position P2.

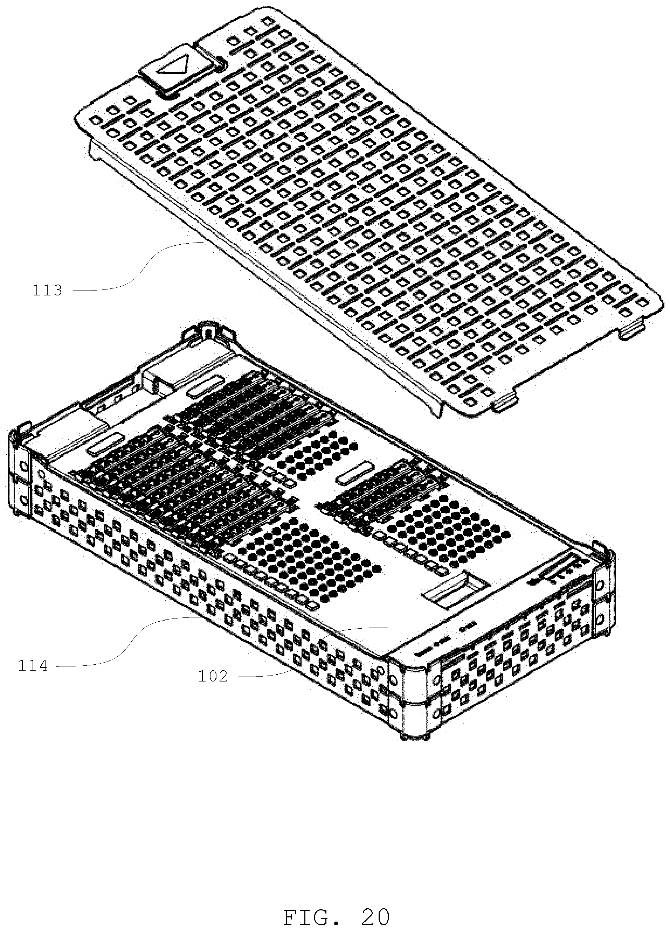

In Figur 20 ist der Container 114 zusammen mit einem Deckel 113 dargestellt, mit dem er verschliessbar ist.

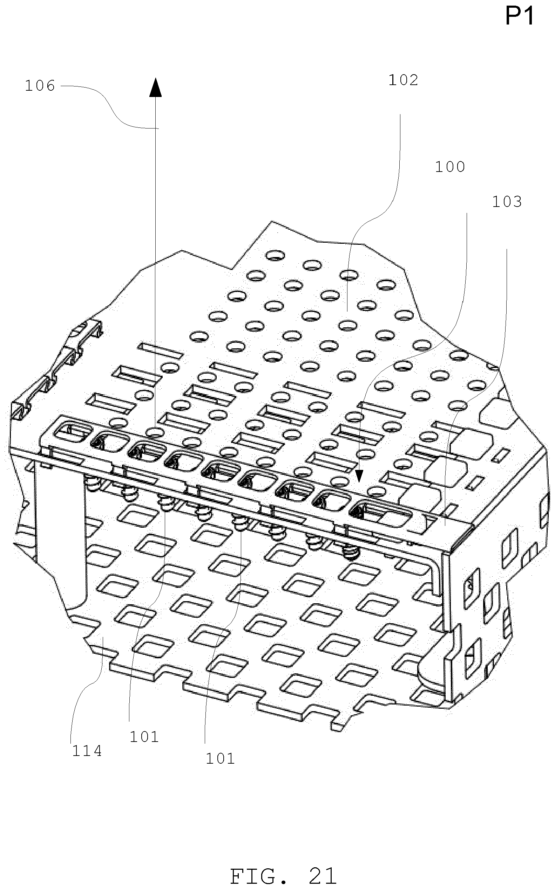

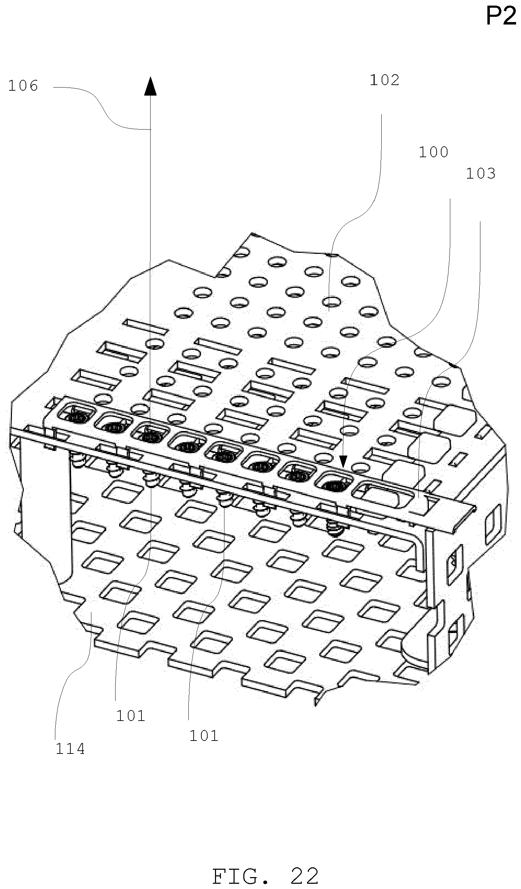

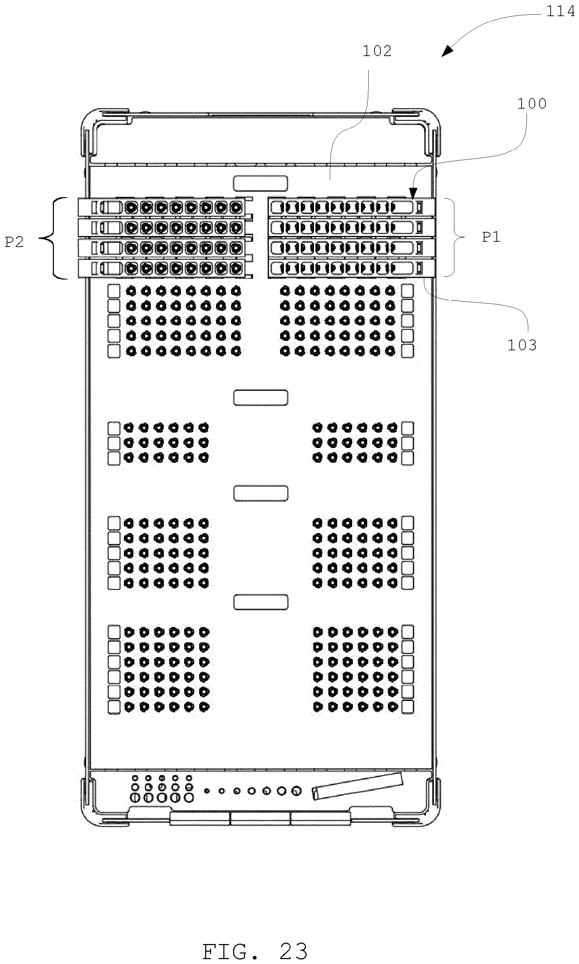

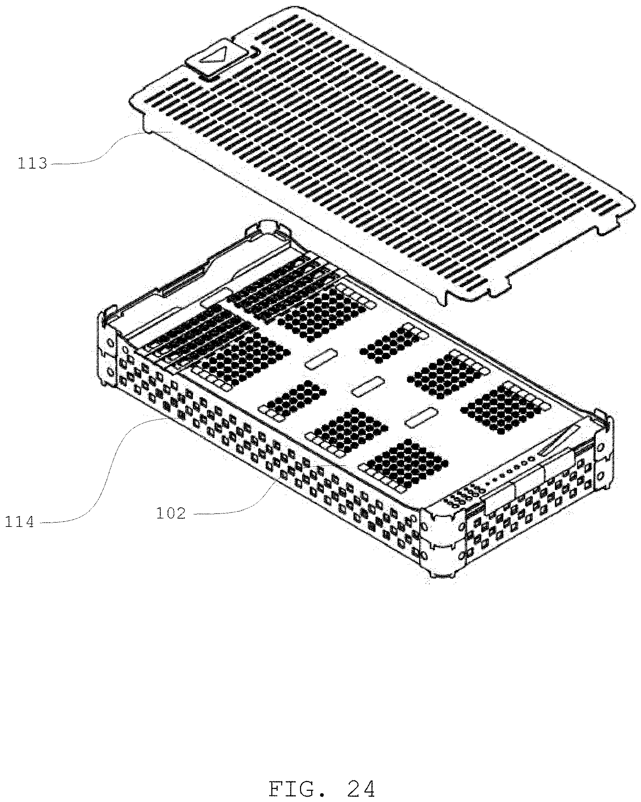

Die Figuren 21 bis 24 zeigen ein weiteres Ausführungsbeispiel. Die Sicherungselemente 103 der in Figur 23 links dargestellten Träger 100 befinden sich in der zweiten Position P2, in der die Sicherungselement 103 über den Rand des Containers 114 hervorstehen. Die Sicherungselemente 103 der in Figur 23 rechts dargestellten Träger 100 befinden sich in der ersten Position P1 und stehen nicht über den Rand des Containers 114 hervor. Solange sich mindestens eines der Sicherungselemente 103 in der zweiten Position P2 befindet, kann kein Deckel 113 auf den Container 114 gesetzt werden. Dies ist nur möglich, wenn die Sicherungselemente 103 in die erste Position P1 überführt werden, in der sie nicht über den Rand des Containers 114 hervorstehen. Somit sind die Schrauben 101 bei aufgesetztem Deckel 113 stets gegen ein Herausfallen gesichert.

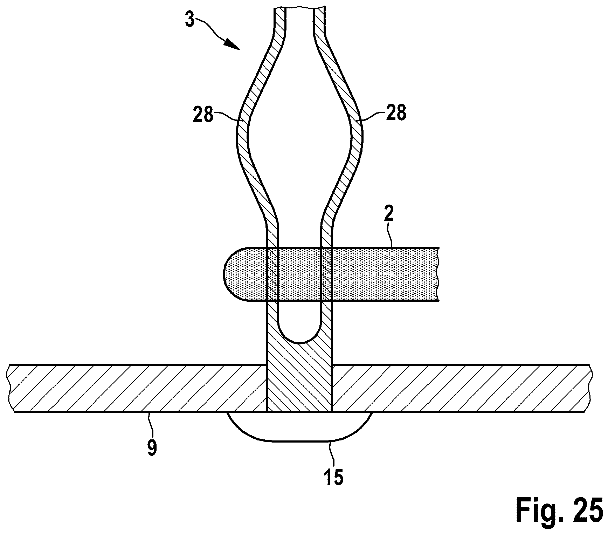

Der in Figur 25 gezeigte beispielhafte Träger 1 für eine Knochenplatte 2 enthält zwei Blattfedern 28, die sich im Wesentlichen entlang der gemeinsamen Längsachse 4 erstrecken, die jedoch in einem mittleren Bereich einen grösseren Abstand voneinander aufweisen. Wird eine Knochenplatte 2 mit einer Ausnehmung über die Blattfedern 28 gesteckt, so werden die Blattfedern 28 gegeneinander gedrückt und die Knochenplatte 2 kann über den mittleren Bereich geschoben werden. Nachdem die Knochenplatte 2 den mittleren Bereich passiert hat, federn die Blattfedern 28 wieder auseinander und sichern somit die Knochenplatte 2 gegen ein unbeabsichtigtes Herausfallen.

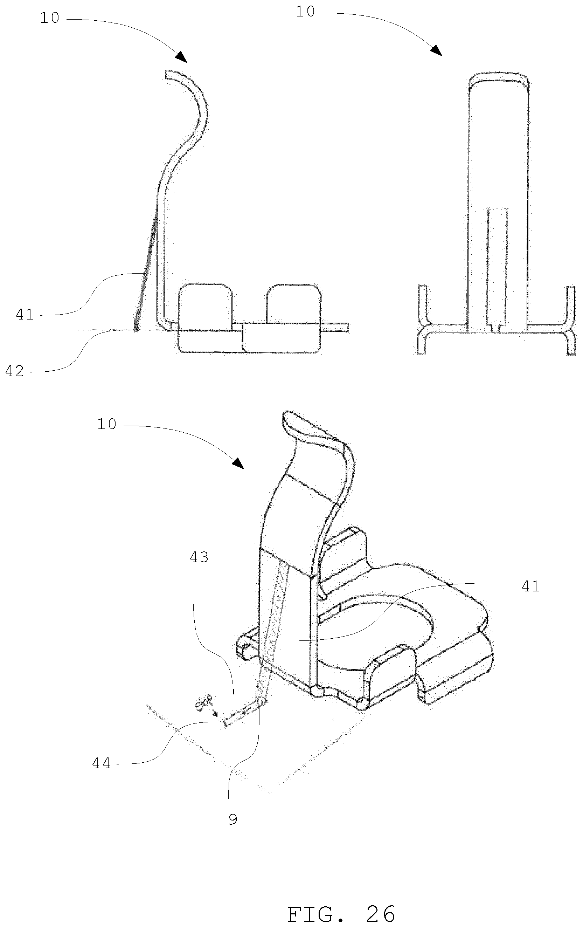

Figur 26 zeigt eine Feder 10 eines weiteren beispielhaften Trägers 1. Die Feder 10 verfügt über eine Lasche 41 mit einem Ende 42, dass in einer Schiene 43 geführt ist. Ein Ende 44 der Schiene 43 bildet einen Anschlag, der ein übermässiges Verbiegung der Feder 10 verhindert.

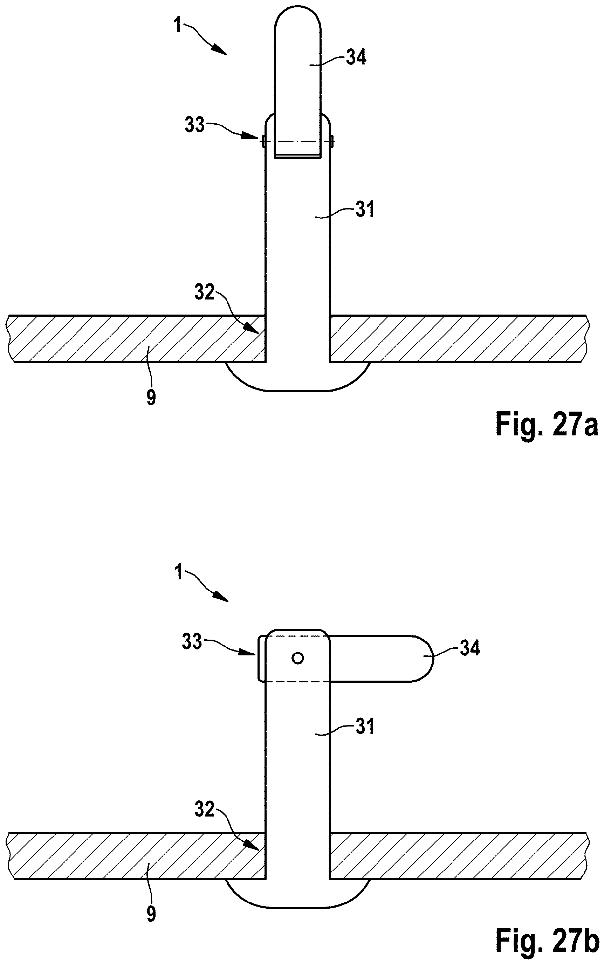

In den Figuren 27a und 27b ist ein weiterer Träger 1 für eine Knochenplatte dargestellt. Der Träger 1 enthält einen Schaft 31 zur Aufnahme der Knochenplatte an einer ihrer Ausnehmungen. Der Schaft 31 weist an einem ersten, hier unteren Ende 32 Mittel zur Befestigung an einem Halteabschnitt 9 eines Container auf. Weiterhin enthält der Träger 1 ein Riegelelement 34, welches schwenkbar an einem dem ersten Ende 32 des Schafts 31 gegenüberliegenden zweiten, hier oberen Ende 33 des Schafts 31 angeordnet ist. Das Riegelelement 34 kann in eine in Figur 27a gezeigte Aufnahmeposition und in eine in Figur 27b gezeigte Verriegelungsposition geschwenkt werden. In der Aufnahmeposition kann die Knochenplatte mit der Ausnehmung über das Riegelelement 34 auf den Schaft 31 bewegt werden. Durch Schwenken des Riegelelements 34 in die Verriegelungsposition verhindert das Riegelelement 34 die Entnahme der Knochenplatte.

## Patentansprüche

1. Träger (100) zur Aufnahme zumindest eines chirurgischen Gegenstandes, insbesondere eines Implantats, insbesondere einer Schraube (101), enthaltend:
- eine Halterung (102) mit zumindest einer Aufnahme (104) für den zumindest einen chirurgischen Gegenstand,
- ein Sicherungselement (103) für den zumindest einen Gegenstand,
wobei
- das Sicherungselement (103) zumindest eine Ausnehmung (105) zur Entnahme des zumindest einen Gegenstandes durch die Ausnehmung (105) aufweist,
- das Sicherungselement (103) bewegbar mit der Halterung (102) verbunden ist, so dass das Sicherungselement (103) von einer ersten Position (P1) in eine zweite Position (P2) bewegbar ist,
- in der ersten Position (P1) des Sicherungselements (103) relativ zur Halterung (102) der zumindest eine Gegenstand gesichert ist und
- in der zweiten Position (P2) des Sicherungselements relativ zur Halterung (102) der zumindest eine Gegenstand aus der Aufnahme (104) durch die Ausnehmung (105) des Sicherungselements (103) entlang einer Entnahmerichtung (106) entnehmbar ist.

2. Träger (100) nach Anspruch 1, wobei der Träger (100) in einen Container (114) eingesetzt ist, der mit einem Deckel (113) verschliessbar ist, und wobei das Sicherungselement (103) beim Schliessen des Deckels (113) derart mit dem Deckel (113) in Wirkverbindung ist oder bringbar ist, dass das Sicherungselement (103) durch das Schliessen des Deckels (113) in die erste Position (P1) gebracht wird.

3. Träger (100) gemäss einem der Ansprüche 1 und 2, wobei die Halterung (102) zwei oder drei oder mehr Aufnahmen (104) für chirurgische Gegenstände (101) aufweist und wobei das Sicherungselement (103) zu den Aufnahmen (104) korrespondierende Ausnehmungen (105) aufweist, wobei die chirurgischen Gegenstände (101) in der ersten Position (P1) in den Aufnahmen (104) durch das Sicherungselement (103) sicherbar sind und in der zweiten Position (P1) durch die je korrespondierenden Ausnehmungen (105) im Sicherungselement (103) entnehmbar sind.

4. Träger (100) gemäss Anspruch 3, wobei die Aufnahmen (104) entlang einer geraden Linie (107) angeordnet sind.

5. Träger (100) gemäss einem der Ansprüche 1 bis 4, wobei der Träger (100), insbesondere das Sicherungselement (103), ein erstes Federelement (108) aufweist, das derart ausgebildet und angeordnet ist, dass das Sicherungselement (103) in der ersten Position (P1) gehalten wird.

6. Träger (100) gemäss Anspruch 5, wobei das erste Federelement (108) in einem ersten Endbereich (109) des Sicherungselements (103) angeordnet ist und bevorzugt einstückig mit dem Sicherungselement (103) ausgebildet ist.

7. Träger (100) gemäss einem der Ansprüche 1 bis 6, wobei der Träger (100) eine Rastvorrichtung (110) aufweist, welche ausgebildet ist, einzurasten und das Sicherungselement (103) in der zweiten Position (P2) zu fixieren, sobald das Sicherungselement (103) die zweite Position (P2) erreicht.

8. Träger (100) gemäss Anspruch 7, wobei die Rastvorrichtung (110) lösbar ist.

9. Träger (100) gemäss einem der Ansprüche 1 bis 8, wobei die Entnahmerichtung (106) im Wesentlichen einer Längsachse eines bestimmungsgemäss in der Ausnehmung (105) gehaltenen Gegenstandes (101) entspricht.

10. Träger (100) gemäss einem der Ansprüche 1 bis 9, wobei das Sicherungselement (103) beschriftet ist, bevorzugt durch eine Lasermarkierung (116).

11. Träger (100) gemäss einem der Ansprüche 1 bis 10, wobei das Sicherungselement (103) zumindest zwei Füsse (111) aufweist, die sich von dem Sicherungselement (103) in Richtung der Halterung (102) erstrecken, wobei die Füsse (111) elastisch ausgebildet sind und wobei die Füsse (111) zumindest teilweise eine Gegenkontur zu einem Teil der Halterung (102) bilden, so dass die Füsse (111) an der Halterung (102) einrastbar sind.

12. Träger (100) gemäss Anspruch 11, wobei die Füsse (111) im eingerasteten Zustand entlang einer Kante (115) der Halterung (102) von der ersten Position (P1) in die zweite Position (P2) verschiebbar sind.

13. Träger (100) gemäss Anspruch 12, wobei die Kanten (115) für die Füsse (111) durch längliche Halterungsausnehmungen (112) in der Halterung (102) gebildet sind.

14. Set aus einem Träger (100) gemäss einem der Ansprüche 1 bis 13 und zumindest einem chirurgischen Gegenstand, insbesondere einem chirurgischen Implantat, insbesondere einer Schraube (101), wobei der chirurgische Gegenstand (101) eine Querschnittsverdickung, bevorzugt einen Kopf, aufweist, welche bevorzugt an einem Rand der Aufnahme (104) aufliegt, wobei das Sicherungselement (103) in der ersten Position (P1) derart angeordnet ist, dass die Querschnittsverdickung blockiert wird und nicht entlang der Entnahmerichtung (106) entnehmbar ist.

15. Container (114) für chirurgische Gegenstände, insbesondere chirurgische Implantate, insbesondere Schrauben (101), enthaltend zumindest einen, bevorzugt mehrere, Träger (100) gemäss einem der Ansprüche 1 bis 13 und/oder Sets gemäss Anspruch 14.

## Claims

1. Carrier (100) for receiving at least one surgical object, in particular an implant, in particular a screw (101):
- a holder (102) with at least one receptacle (104) for the at least one surgical object,
- a securing element (103) for the at least one object, whereby
- the securing element (103) has at least one recess (105) for removing the at least one object through the recess (105),
- the securing element (103) is movably connected to the holder (102), so that the securing element (103) can be moved from a first position (P1) to a second position (P2),
- in the first position (P1) of the securing element (103) relative to the holder (102), the at least one object is secured, and
- in the second position (P2) of the securing element relative to the holder (102), the at least one object can be removed from the receptacle (104) through the recess (105) of the securing element (103) along a removal direction (106).

2. Carrier (100) according to claim 1, wherein the carrier (100) is inserted into a container (114) which can be closed with a lid (113), and wherein the securing element (103) is or can be brought into operative connection with the lid (113) when the lid (113) is closed in such a way that the securing element (103) is brought into the first position (P1) by the closing of the lid (113).

3. Carrier (100) according to one of claims 1 and 2, wherein the holder (102) has two or three or more receptacles (104) for surgical objects (101) and wherein the securing element (103) has recesses (105) corresponding to the receptacles (104), wherein the surgical objects (101) can be secured in the first position (P1) in the receptacles (104) by the securing element (103) and can be removed in the second position (P1) through the respective corresponding recesses (105) in the securing element (103).

4. Carrier (100) according to claim 3, wherein the receptacles (104) are arranged along a straight line (107).

5. Carrier (100) according to one of claims 1 to 4, wherein the carrier (100), in particular the securing element (103), comprises a first spring element (108) which is designed and arranged in such a way that the securing element (103) is held in the first position (P1).

6. Carrier (100) according to claim 5, wherein the first spring element (108) is arranged in a first end region (109) of the securing element (103) and is preferably formed integrally with the securing element (103).

7. The carrier (100) according to any one of claims 1 to 6, wherein the carrier (100) comprises a latching device (110) which is configured to latch and fix the securing element (103) in the second position (P2) as soon as the securing element (103) reaches the second position (P2).

8. Carrier (100) according to claim 7, wherein the latching device (110) is releasable.

9. The carrier (100) according to any one of claims 1 to 8, wherein the removal direction (106) substantially corresponds to a longitudinal axis of an object (101) held in the recess (105) as intended.

10. Carrier (100) according to any one of claims 1 to 9, wherein the securing element (103) is labeled, preferably by a laser marking (116).

11. Support (100) according to one of claims 1 to 10, wherein the securing element (103) has at least two feet (111) which extend from the securing element (103) in the direction of the holder (102), wherein the feet (111) are of elastic design and wherein the feet (111) at least partially form a counter-contour to a part of the holder (102), so that the feet (111) can be latched onto the holder (102) .

12. Carrier (100) according to claim 11, wherein the feet (111) are displaceable along an edge (115) of the holder (102) from the first position (P1) to the second position (P2) in the engaged state.

13. The support (100) according to claim 12, wherein the edges (115) for the feet (111) are formed by elongate support recesses (112) in the support (102).

14. Set comprising a carrier (100) according to one of claims 1 to 13 and at least one surgical object, in particular a surgical implant, in particular a screw (101), wherein the surgical object (101) has a cross-sectional thickening, preferably a head, which preferably rests on an edge of the receptacle (104), wherein the securing element (103) is arranged in the first position (P1) in such a way that the cross-sectional thickening is blocked and cannot be removed along the removal direction (106).

15. Container (114) for surgical objects, in particular surgical implants, in particular screws (101), comprising at least one, preferably several, carriers (100) according to one of claims 1 to 13 and/or sets according to claim 14.

## Revendications

1. Support (100) destiné à recevoir au moins un objet chirurgical, en particulier un implant, notamment une vis (101), contenant :
- un posage (102) comprenant au moins un logement (104) pour ledit au moins un objet chirurgical,
- un élément de sécurisation (103) pour ledit au moins un objet,
dans lequel
- l'élément de sécurisation (103) présente au moins un évidement (105) pour l'enlèvement du au moins un objet à travers l'évidement (105),
- l'élément de sécurisation (103) est relié de manière mobile au posage (102), de sorte que élément de sécurisation (103) peut être déplacé d'une première position (P1) à une seconde position (P2),
- dans la première position (P1) de l'élément de sécurisation (103) par rapport au posage (102), l'au moins un objet est bloqué, et
- dans la deuxième position (P2) de l'élément de sécurisation par rapport au posage (102), le au moins un objet peut être retiré du logement (104) à travers l'évidement (105) de l'élément de sécurisation (103) le long d'une direction de retrait (106).

2. Support (100) selon la revendication 1, dans lequel le support (100) est inséré dans un conteneur (114) qui peut être fermé par un couvercle (113), et dans lequel l'élément de sécurisation (103) est ou peut être amené en liaison active avec le couvercle (113) lors de la fermeture du couvercle (113) de telle sorte que l'élément de sécurisation (103) est amené dans la première position (P1) par la fermeture du couvercle (113).

3. Support (100) selon l'une des revendications 1 et 2, dans lequel le posage (102) présente deux ou trois ou plusieurs logements (104) pour des objets chirurgicaux (101) et dans lequel l'élément de sécurisation (103) présente des évidements (105) correspondant aux logements (104), les objets chirurgicaux (101) pouvant être fixés dans la première position (P1) dans les logements (104) par l'élément de sécurisation (103) et pouvant être retirés dans la deuxième position (P1) par les évidements (105) correspondant respectivement dans l'élément de sécurisation (103).

4. Support (100) selon la revendication 3, dans lequel les logements (104) sont disposés le long d'une ligne droite (107) .

5. Support (100) selon l'une des revendications 1 à 4, dans lequel le support (100), notamment l'élément de sécurisation (103), comporte un premier élément de ressort (108) configuré et agencé de manière à maintenir l'élément de sécurisation (103) dans la première position (P1).

6. Support (100) selon la revendication 5, dans lequel le premier élément de ressort (108) est disposé dans une première zone d'extrémité (109) de l'élément de sécurisation (103) et est de préférence formé d'une seule pièce avec l'élément de sécurisation (103).

7. Support (100) selon l'une des revendications 1 à 6, dans lequel le support (100) comporte un dispositif d'encliquetage (110) configuré pour s'encliqueter et fixer l'élément de sécurisation (103) dans la deuxième position (P2) dès que l'élément de sécurisation (103) atteint la deuxième position (P2).

8. Support (100) selon la revendication 7, dans lequel le dispositif d'encliquetage (110) est libérable.

9. Support (100) selon l'une des revendications 1 à 8, dans lequel la direction de retrait (106) correspond sensiblement à un axe longitudinal d'un objet (101) maintenu dans l'évidement (105) conformément à sa destination.

10. Support (100) selon l'une des revendications 1 à 9, dans lequel l'élément de sécurisation (103) est marqué, de préférence par un marquage laser (116).

11. Support (100) selon l'une quelconque des revendications 1 à 10, dans lequel l'élément de sécurisation (103) comprend au moins deux pieds (111) qui s'étendent depuis l'élément de sécurisation (103) en direction du posage (102), dans lequel les pieds (111) sont élastiques et dans lequel les pieds (111) forment au moins partiellement un contour opposé à une partie du posage (102), de sorte que les pieds (111) peuvent être encliquetés sur le posage (102).

12. Support (100) selon la revendication 11, dans lequel les pieds (111), à l'état engagé, peuvent être déplacés le long d'un bord (115) du posage (102) de la première position (P1) à la deuxième position (P2).

13. Support (100) selon la revendication 12, dans lequel les bords (115) pour les pieds (111) sont formés par des évidements de maintien allongés (112) dans le posage (102).

14. Ensemble constitué d'un support (100) selon l'une des revendications 1 à 13 et d'au moins un objet chirurgical, en particulier un implant chirurgical, notamment une vis (101), l'objet chirurgical (101) présentant un épaississement de section, de préférence une tête, qui repose de préférence sur un bord du logement (104), l'élément de sécurisation (103) étant disposé dans la première position (P1) de telle sorte que l'épaississement de section soit bloqué et ne puisse pas être retiré le long de la direction de retrait (106).

15. Conteneur (114) pour objets chirurgicaux, en particulier des implants chirurgicaux, notamment des vis (101), contenant au moins un, de préférence plusieurs, support(s) (100) selon l'une des revendications 1 à 13 et/ou des ensembles selon la revendication 14.
